# EUROPEAN PATENT SPECIFICATION

(11) **EP 1 991 221 B1**
(45) Date of publication and mention of the grant of the patent: **21.05.2014**
(21) Application number: 06721002.1
(22) Date of filing: 24.02.2006
(51) Int. Cl.: A61K 31/395, A61P 9/10

(54) **METHODS FOR INCREASING BLOOD FLOW AND/OR PROMOTING TISSUE REGENERATION**
VERFAHREN ZUR ERHÖHUNG DES BLUTFLUSSES UND/ODER ZUR FÖRDERUNG DER GEWEBEREGENERATION
PROCÉDÉS SERVANT À ACCROÎTRE LE DÉBIT SANGUIN ET/OU À FAVORISER LA RÉGÉNÉRATION D'UN TISSU

(43) Date of publication of application: 19.11.2008
(73) Proprietor: Genzyme Corporation, Cambridge, MA 02142-1108 (US)
(72) Inventor: BRIDGER, Gary, J., Bellingham, Washington 98225 (US); FRICKER, Simon, P., Langley, British Columbia, V2Z 1A6 (CA)
(74) Representative: Keen, Celia Mary
(86) International application number: PCT/US2006/006435
(87) International publication number: WO 2007/106063

(56) References cited:
- WO-A2-03/011277
- WO-A2-2005/002522
- WO-A2-2005/017160
- US-A1- 2006 035 829
- US-B1- 6 365 583

## Description

### Technical Field

The invention is in the field of therapeutics and medicinal chemistry. More particularly, the invention concerns methods for increasing blood flow and/or promoting tissue regeneration.

### Background Art

Blood cells play a crucial part in maintaining the health and viability of animals, including humans. White blood cells include neutrophils, macrophage, eosinophils and basophils/mast cells as well the B and T cells of the immune system. White blood cells are continuously replaced via the hematopoietic system, by the action of colony stimulating factors (CSF) and various cytokines on stem cells and progenitor cells in hematopoietic tissues. The nucleotide sequences encoding a number of these growth factors have been cloned and sequenced. Perhaps the most widely known of these is granulocyte colony stimulating factor (G-CSF) which has been approved for use in counteracting the negative effects of chemotherapy by stimulating the production of white blood cells and progenitor cells (peripheral blood stem cell mobilization). A discussion of the hematopoietic effects of this factor can be found, for example, in U.S. Patent No. 5,582,823.

Several other factors have been reported to increase white blood cells and progenitor cells in both human and animal subjects. These agents include granulocyte-macrophage colony stimulating factor (GM-CSF), Interleukin-1 (IL-1), Interleukin-3 (IL-3), Interleukin-8 (IL-8), PIXY-321 (GM-CSF/IL-3 fusion protein), macrophage inflammatory protein, stem cell factor, thrombopoietin and growth related oncogene, as single agents or in combination (Dale, D., et al., Am. J. of Hematol. (1998) 57:7-15; Rosenfeld, C., et al., Bone Marrow Transplantation (1997) 17:179-183; Pruijt, J., et al., Cur. Op. in Hematol. (1999) 6:152-158; Broxmeyer, H., et al., Exp. Hematol. (1995) 23:335-340; Broxmeyer, et al., Blood Cells, Molecules and Diseases (1998) 24:14-30; Glaspy, J., et al., Cancer Chemother. Pharmacol. (1996) 38 (suppl): S53-S57; Vadhan-Raj, S., et al., Ann. Intern. Med. (1997) 126:673-81; King, A., et al., Blood (2001) 97:1534-1542; Glaspy, J., et al., Blood (1997) 90:2939-2951).

While endogenous growth factors are pharmacologically effective, the well known disadvantages of employing proteins and peptides as pharmaceuticals underlies the need to add to the repertoire of such growth factors with agents that are small molecules. In another aspect, such small molecules are advantageous over proteins and peptides where production in large quantities are desired.

A number of cyclic polyamine antiviral agents have been described in a series of U.S. patents and applications (See e.g., U.S. Patent Nos. 5,021,409; 6,001,826; 5,583,131; 5,698,546; and 5,817,807).

WO 00/02870 and WO 01/44229 describe additional compounds and also describe the structural characteristics of the cyclic polyamine antiviral agents. The structural characteristics of a number of non-cyclic amine antiviral agents have also been described in WO 00/56729, WO 02/22600, WO 02/22599, and WO 02/34745.

Improved methods for preparation of some of the cyclic polyamine compounds have also been described in U.S. Patent Nos. 5,612,478; 5,756,728; 5,801,281; and 5,606,053 and PCT publication WO 02/26721.

PCT publication WO 02/58653 teaches that some of the polyamine antiviral agents described in the above mentioned publications have the effect of increasing the white blood cell count. WO 03/011277, US 2006/0035829 and U.S. patent publication US 2005/0043367 teach that some polyamine antiviral agents described in the above-mentioned publications also have the effect of increasing progenitor cells and/or stem cells.

The development and maturation of blood cells is a complex process. Mature blood cells are derived from hematopoietic precursor cells (progenitor) cells and stem cells present in specific hematopoietic tissues including bone marrow. Within these environments hematopoietic cells proliferate and differentiate prior to entering the circulation. The chemokine receptor CXCR4 and its natural ligand stromal cell derived factor-1 (SDF-1) appear to be important in this process (for reviews see Maekawa, T., et al., Internal Med. (2000) 39:90-100; Nagasawa, T., et al., Int. J. Hematol. (2000) 72:408-411). This is demonstrated by reports that CXCR4 or SDF-1 knock-out mice exhibit hematopoietic defects (Ma, Q., et al., Proc. Natl. Acad. Sci USA (1998) 95:9448-9453; Tachibana, K., et al., Nature (1998) 393:591-594; Zou, Y-R., et al., Nature (1998) 393:595-599). It is also known that CD34+ progenitor cells express CXCR4 and require SDF-1 produced by bone marrow stromal cells for chemoattraction and engraftment (Peled, A., et al., Science (1999) 283:845-848) and that *in vitro,* SDF-1 is chemotactic for both CD34+ cells (Aiuti, A., et al., J. Exp. Med. (1997) 185:111-120; Viardot, A., et al., Ann. Hematol. (1998) 77:194-197) and for progenitor/stem cells (Jo, D-Y., et al., J. Clin. Invest. (2000) 105:101-111). SDF-1 is also an important chemoattractant, signaling via the CXCR4 receptor, for several other more committed progenitors and mature blood cells including T-lymphocytes and monocytes (Bleul, C., et al., J. Exp. Med. (1996) 184:1101-1109), pro-and pre-B lymphocytes (Fedyk, E. R., et al., J. Leukoc. Biol. (1999) 66:667-673; Ma, Q., et al., Immunity (1999) 10:463-471) and megakaryocytes (Hodohara, K., et al., Blood (2000) 95:769-775; Riviere, C., et al., Blood (1999) 95:1511-1523; Majka, M., et al., Blood (2000) 96:4142-4151; Gear, A., et al., Blood (2001) 97:937-945; Abi-Younes, S., et al., Circ. Res. (2000) 86:131-138).

Thus, in summary, it appears that SDF-1 is able to control the positioning and differentiation of cells bearing CXCR4 receptors whether these cells are stem cells (*i.e.,* cells which are CD34+) and/or progenitor cells (which result in formation of specified types of colonies in response to particular stimuli; that can be CD34⁺ or CD34⁻) or cells that are somewhat more differentiated.

Considerable attention has been focused on the number of CD34+ cells mobilized in the pool of peripheral blood progenitor cells used for autologous stem cell transplantation. The CD34+ population is the component thought to be primarily responsible for the improved recovery time after chemotherapy and the cells most likely responsible for long-term engraftment and restoration of hematopoiesis (Croop, J. M., et al., Bone Marrow Transplantation (2000) 26:1271-1279). The mechanism by which CD34+ cells re-engraft may be due to the chemotactic effects of SDF-1 on CXCR4 expressing cells (Voermans, C. Blood, 2001, 97, 799-804; Ponomaryov, T., et al., J. Clin. Invest. (2000) 106:1331-1339). More recently, adult hematopoietic stem cells were shown to be capable of restoring damaged cardiac tissue in mice (Jackson, K., et al., J. Clin. Invest. (2001) 107:1395-1402; Kocher, A., et al., Nature Med. (2001) 7:430-436).

There is growing evidence to show support for the concept of stem cell plasticity and the use of progenitor and/or stem cells, particularly stem cells in tissue repair (Orlic et al., Circ. Res. (2002 91:1092-1102)). Thus, the role of the CXCR4 receptor in managing cell positioning and differentiation has assumed considerable significance.

Citation of the above documents is not intended as an admission that any of the foregoing is pertinent prior art. All statements as to the date or representation as to the contents of these documents is based on the information available to the applicants and does not constitute any admission as to the correctness of the dates of the dates or contents of these documents.

### Disclosure of the Invention

The invention is directed to methods of increasing blood flow in a subject, particularly a veterinary or human subject, comprising directly administering mobilized and harvested progenitor and/or stem cells to target tissues in a subject.

The present invention therefore provides the use of a compound having formula (1) for the manufacture of a medicament for increasing blood flow in an ischemic tissue, wherein said compound having formula (1) mobilizes progenitor and/or stem cells, which are harvested and directly administered to an ischemic tissue in a subject, wherein said compound is of the formula:

Z-linker-Z' (1)

or a pharmaceutically acceptable salt thereof,
wherein Z is a cyclic polyamine containing 9-32 ring members of which 2-8 are nitrogen atoms, said nitrogen atoms separated from each other by at least 2 carbon atoms, and wherein said heterocycle may optionally contain additional heteroatoms besides nitrogen and/or may be fused to an additional ring system;
Z' may be embodied in a form as defined by Z above, or alternatively may be of the formula:

-N(R)-(CR₂)ₙ-X

wherein each R is independently H or straight, branched or cyclic alkyl (1-6C),
n is 1 or 2, and
X is an aromatic ring, including heteroaromatic rings, or is a mercaptan;
"linker" represents a bond, alkylene (1-6C) or may comprise aryl, fused aryl, oxygen atoms contained in an alkylene chain or may contain keto groups or nitrogen or sulphur atoms.

In one embodiment, the progenitor and/or stem cells in the methods of the invention are mobilized and harvested by apheresis. In other embodiments, the progenitor and/or stem cells are mobilized into the peripheral blood or bone marrow of said subject and harvested. In some examples, the peripheral blood or bone marrow is derived from a subject who has been treated with granulocyte colony-stimulating factor. In other examples, the progenitor and/or stem cells are harvested from bone marrow.

The ischemic tissue may be an organ tissue or a limb tissue. Examples of organ tissue include but are not limited to myocardium, brain tissue, lung tissue, or liver tissue.

In particular examples, the progenitor and/or stem cells are mobilized by 1,1'-1,4-phenylene-bis-(methylene)-bis-1,4,8,11-tetraazacyclotetradecane (AMD 3100) or a pharmaceutically acceptable salt thereof. For example, the progenitor and/or stem cells may be mobilized by an acid addition salt of AMD 3100, such as AMD 3100 hydrochloride.

In the present invention, the compound of formula (1) may be directly administered to damaged tissues in said subject in a dosage range of about 0.1 µg/kg-5mg/kg of body weight. For example, the compound of formula (1) may be directly administered in the dosage range of about 1 µg/kg to 300 µg/kg of body weight, or in the dosage range of about 10 µg/kg to 100 µg/kg of body weight.

In the present invention, the subject may be a diabetic subject, or a human subject that is suffering or has suffered from limb ischemia or a peripheral vascular disease, such as a peripheral arterial disease, or peripheral venous disorders. Examples of peripheral arterial disease include but are not limited to atherosclerosis, carotid artery disease, peripheral arterial disease of the legs, peripheral arterial disease of the renal arteries, abdominal aortic aneurysm, Raynaud syndrome, Buerger disease, or vasculitis.

The present invention also includes treatment of cell populations *ex vivo* with a compound of formula (1) and directly administering the treated populations into damaged tissues in a compatible subject. The compounds of formula (1) may be used alone or in combination with other compounds and compositions to enhance the population of stem cells and/or progenitor cells in the peripheral blood. An enhanced production of white blood cells in the bone marrow may result as well.

### Brief Description of the Drawings

Figure 1 shows a graph of obtaining myeloid progenitors in response to treatment with 1,1'-[1,4-phenylene-bis(methylene)-bis-1,4,8,11-tetraazacyclotetradecane (AMD 3100) in combination with macrophage inflammatory protein after administration of granulocyte colony-stimulating factor (G-CSF).

Figure 2 shows the percent of blood flow restoration in ischemic limbs followed by A) systemic administration of AMD 3100 in mice; and B) local injection of harvested CD34+ cells mobilized by AMD 3100.

### Modes of Carrying Out the Invention

The compounds useful in the invention are of the general formula set forth as formula (1) above, Certain embodiments are preferred; included among these are the compounds set forth in the above-incorporated U.S. patents and other patent documents. In particular examples, the compounds for use in the methods of the invention comprise AMD 3100.

In one embodiment, the compounds useful in the methods of the invention comprise cyclic polyamine antiviral agents. As described in the above-mentioned documents, cyclic polyamine antiviral agents inhibit HIV replication via inhibition of CXCR4, the co-receptor required for fusion and entry of T-tropic HIV strains, and also inhibit the binding and signalling induced by the natural ligand, the chemokine SDF-1. While not wishing to be bound by any theory, the compounds of formula (1) which inhibit the binding of SDF-1 to CXCR4 effect an increase in stem and/or progenitor cells by virtue of such inhibition. Enhancing the stem and/or progenitor cells in blood is helpful in treatments to alleviate the effects of protocols that adversely affect the bone marrow, such as those that result in leukopenia. These are known side-effects of chemotherapy and radiotherapy.

The compounds useful for the methods of the invention also enhance the success of bone marrow transplantation, enhance wound healing and bum treatment, and aid in restoration of damaged organ tissue. They also combat bacterial infections that are prevalent in leukemia. The compounds useful for the methods of the invention are used to mobilize and harvest CD34+ cells via apheresis with and without combinations with other mobilizing factors. The harvested cells are used in treatments requiring stem cell transplantations.

In one embodiment, progenitor and/or stem cells are mobilized by polyamine compounds, harvested and directly administered to target tissues in a subject that is in need of increased blood flow, particularly damaged tissues. For example, the harvested mobilized cells may be directly injected into cardiac tissue, neural tissue, ischemic tissue, or post-ischemic tissue. In other embodiments, mobilized and harvested progenitor and/or stem cells are systematically administered to the subject (e.g., subcutaneously or intraperitoneal administration). In yet other embodiments, mobilized and harvested progenitor and/or stem cells are administered directly to the heart muscle, left ventricle, right ventricle, coronary artery, peripheral circulation, or cerebro-spinal fluid.

In another aspect, the progenitor and/or stem cells may be further separated by selective purification to isolate specific cell populations. The homogenous extracted cells may than be administered as described in this invention.

As used herein, the term "progenitor cells" refers to cells that, in response to certain stimuli, can form differentiated hematopoietic or myeloid cells. The presence of progenitor cells can be assessed by the ability of the cells in a sample to form colony-forming units of various types, including, for example, CFU-GM (colony-forming units, granulocyte-macrophage); CFU-GEMM (colony-forming units, multipotential); BFU-E (burst-forming units, erythroid); HPP-CFC (high proliferative potential colony-forming cells); or other types of differentiated colonies which can be obtained in culture using known protocols.

As used herein, "stem" cells are less differentiated forms of progenitor cells. Typically, such cells are often positive for CD34. Some stem cells do not contain this marker, however. These CD34+ cells can be assayed using fluorescence activated cell sorting (FACS) and thus their presence can be assessed in a sample using this technique.

As used herein, "tissue" cells include but are not limited to cardiac tissue, brain tissue, peripheral vascular tissue, hepatic tissue, renal tissue, gastrointestinal tissue, lung tissue, liver tissue, smooth muscle tissue, or striated muscle tissue. As used herein, "damaged tissues" encompass any tissue in need of tissue regeneration, including but not limited to damaged tissues in need of increased blood flow. Tissues may be damaged as a result of a disease, such as heart disease and stroke.

As used herein, "peripheral vascular disease" refers to damage or dysfunction wtihin peripheral arteries and veins.

In general, CD34+ cells are present only in low levels in the blood, but are present in large numbers in bone marrow. While other types of cells such as endothelial cells and mast cells also may exhibit this marker, CD34 is considered an index of stem cell presence.

In general, in compounds of formula (1), preferred embodiments of Z and Z' are cyclic polyamine moieties having from 9-24C that include 3-5 nitrogen atoms. Particularly preferred are 1,5,9,13-tetraazacyclohexadecane; 1,5,8,11,14-pentaazacyclohexadecane; 1,4,8,11-tetraazacylotetradecane; 1,5,9-triazacyclododecane; 1,4,7,10-tetraazacyclododecane; and the like, including such cyclic polyamines which are fused to an additional aromatic or heteroaromatic rings and/or containing a heteroatom other than nitrogen incorporated in the ring. Embodiments wherein the cyclic polyamine contains a fused additional cyclic system or one or more additional heteroatoms are described in U.S. Patent No. 5,698,546 and WO 01/44229 incorporated hereinabove by reference. Also preferred are
3,7,11,17-tetraazabicyclo(13.3.1)heptadeca-1(17),13,15-triene;
4,7,10,17-tetraazabicyclo(13.3.1)heptadeca-1(17),13,15-triene;
1,4,7,10-tetraazacyclotetradecane; 1,4,7-triazacyclotetradecane; and
4,7,10-triazabicyclo(13.3.1)heptadeca-1(17),13,15-triene.

When Z' is other than a cyclic polyamine as defined in Z, its preferred embodiments are set forth in U.S. Patent No. 5,817,807, also incorporated herein by reference.

In one embodiment, each "R" group in the compounds useful for the methods of the invention is independently straight or branched chain alkyl or may be cyclic, and may optionally be substituted by 1-2 substituents selected from halo, hydroxy and alkoxy. Preferably each R is H or lower straight-chain alkyl (1-4C), preferably methyl.

In the compounds of formula (1), Ar may be the residue of an aromatic or heteroaromatic moiety which contains a single or fused ring system and containing 5-6 ring members in the monocyclic system and 9-12 members in the fused ring system. The residue may be optionally substituted. Examples of optionally substituted aromatic and heteroaromatic groups include benzene, naphthalene, dihydronaphthalene, tetrahydronaphthalene, pyridine, quinoline, isoquinoline, imidazole, benzimidazole, azabenzimidazole, benzotriazole, furan, benzofuran, thiazole, benzothiazole, oxazole, benzoxazole, pyrrole, indole, imidazole, tetrahydroquinoline, tetrahydroisoquinoline, pyrazole, thiophene, isoxazole, isothiazole, triazole, tetrazole, oxadiazole, thiadiazole, imidazoline, and benzopyran. Oxides of the nitrogen and sulfur containing heteroaromatic rings are also included in the present invention. Particularly preferred forms of Ar are phenylene, pyridylene or pyridinylene.

When compounds of formula (1) contain elements that are "optionally substituted" these substituents are preferably halogen, nitro, cyano, carboxylic acid, optionally substituted alkyl, alkenyl or cycloalkyl groups, an optionally substituted hydroxyl group, an optionally substituted thiol group, an optionally substituted amino, an optionally substitute acyl group, an optionally substituted carboxylate, carbamate, carboxamide or sulfonamide group, or an optionally substituted aromatic or heterocyclic group.

Examples of halogen include fluorine, chlorine, bromine, iodine, etc., with fluorine and chlorine preferred.

Examples of optionally substituted alkyl include C₁₋₁₀ alkyl, including methyl, ethyl propyl, etc.; examples of optionally substituted alkenyl groups include C₂₋₁₀ alkenyl such as allyl, crotyl, 2-pentenyl, 3-hexenyl, etc.; and examples of optionally substituted cycloalkyl groups include C₃₋₁₀ cycloalkyl such as cyclopropyl, cyclobutyl, cyclopentyl, cyclohexyl, cycloheptyl, etc. In these cases, C₁₋₆ alkyl, alkenyl and cycloalkyl are preferred. The optional substituent may also be an optionally substituted aralkyl (*e.g.*, phenyl C₁₋₄ alkyl) or heteroalkyl for example, phenylmethyl (benzyl), phenylethyl, pyridinylmethy, pyridinylethyl, etc. The heterocyclic group may be a 5 or 6 membered ring containing 1-4 heteroatoms.

Particularly preferred embodiments of the compound of the formula (1) include 2,2'-bicyclam; 6,6'-bicyclam; the embodiments set forth in U.S. Patent Nos. 5,021,409, and 6,001,826, and in particular 1,1'-[1,4-phenylene-bis(methylene)]-bis-1,4,8,11-tetraazacyclotetradecane, set forth in U.S. Patent No. 5,583,131, and designated herein AMD3100.

Other preferred embodiments include
N-[1,4,8,11-tetraazacyclotetradecanyl-1,4-phenylenebis(methylene)]-2-aminomethyl)pyridine;
7,7'-[1,4-phenylenebis(methylene)]bis-4,7,10,17-tetraazabicyclo-[13.3.1] heptadeca-1(17),13,15-triene;
7,7'-[1,4-phenylenebis(methylene)]bis-3,7,11,17-tetraazabicyclo[13.3.1] heptadeca-1(17),13,15-triene;
1,1'-[1,3-phenylenebis(methylene)]-bis-1,4,8,11-tetra-azacyclotetradecane;
1,1'-[1,4-phenylenebis(methylene)]-bis-1,4,8,11-tetra-azacyclotetradecane;
1,1'-[1,4-phenylene-bis-(methylene)]-bis-1,4,7,10-tetraazacyclotetradecane;
1,1'-[1,3-phenylene-bis-(methylene)]-bis-1,4,7,10-tetraazacyclotetradecane;
11,11'-(1,2-propanediyl)bis-1,4,8,11-tetraazacyclotetradecane;
N-[4-(1,4,7-triazacyclotetra-decane)-1,4-phenylenebis(methylene)]-2-(aminomethyl)pyridine;
N-[7-(4,7,10-triazabicyclo[13.3.1]heptadeca-1(17),13,15-triene)-1,4-phenylenebis(methylene)]-2-(aminomethyl)pyridine;
N-[7-(4,7,10,17-tetraazabicyclo[13.3.1]heptadeca-1(17),13,15-triene)-1,4-phenylenebis(methylene)]-2-(aminomethyl)pyridine;
N-[4-[4,7,10,17-tetraazabicyclo[13.3.1]heptadeca-1(17),13,15-triene]-1,4-phenylenebis(methylene)]-2-(aminomethyl)pyridine;
3,3'-(bis-1,5,9,13-tetraazacyclohexadecane);
3,3'-(bis-1,5,8,11,14-pentaazacyclohexadecane), methylene (or polymethylene) di-1-N-1,4,8,11-tetraazacyclotetradecane;
3,3'-bis-1,5,9,13,-tetraazacyclohexadecane;
3,3'-bis-1,5,8,11,14-pentaazacyclohexadecane;
5,5'-bis-1,4,8,11-tetraazacyclotetradecane;
2,5'-bis-1,4,8,11-tetraazacyclotetradecane;
2,6'-bis-1,4,8,11-tetraazacyclotetradecane;
11,11'-(1,2-ethanediyl)bis-1,4,8,11-tetraazacyclotetradecane;
11,11'-(1,2-propanediyl)bis-1,4,8,11-tetraazacyclotetradecane;
11,11'-(1,2-butanediyl)bis-1,4,8,11-tetraazacyclotetradecane;
11,11'-(1,2-pentanediyl)bis-1,4,8,11-tetraazacyclotetradecane;
11,11'-(1,2-hexanediyl)bis-1,4,8,11-tetraazacyclotetradecane;
3,3'-bis-1,5,9,13-tetraazacyclohexadecane;
3,3'-bis-1,5,8,11,14-pentaazacyclohexadecane;
5,5'-bis-1,4,8,11-tetraazacyclotetradecane;
2,5'-bis-1,4,8,11-tetraazacyclotetradecane;
2,6'-bis-1,4,8,11-tetraazacyclotetradecane;
11,11'-(1,2-ethanediyl)bis-1,4,8,11-tetraazacyclotetradecane;
11,11'-(1,2-propanediyl)bis-1,4,8,11-tetraazacyclotetradecane;
11,11'-(1,2-butanediyl)bis-1,4,8,11-tetraazacyclotetradecane;
11,11'-(1,2-pentanediyl)bis-1,4,8,11-tetraazacyclotetradecane;
11,11'-(1,2-hexanediyl)bis-1,4,8,11-tetraazacyclotetradecane;
1,1'-[1,3-phenylenebis(methylene)]-bis-1,4,8,11-tetra-azacyclotetradecane;
1,1'-[1,4-phenylenebis(methylene)]-bis-1,4,8,11-tetra-azacyclotetradecane ;
1,1'-[3,3'-biphenylene-bis-(methylene)]-bis-1,4,8,11-tetraazacyclotetradecane;
11,11'-[1,4-phenylene-bis-(methylene)]-bis-1,4,7,11-tetraazacyclotetradecane;
1,11'-[1,4-phenylene-bis(methylene)]-1,4,8,11-tetraazacyclotetradecane;
1,1'-[2,6-pyridine-bis-(methylene)]-bis-1,4,8,11-tetraazacyclotetradecane;
1,1-[3,5-pyridine-bis-(methylene)]-bis-1,4,8,11-tetraazacyclotetradecane;
1,1'-[2,5-thiophene-bis-(methylene)]-bis-1,4,8,11-tetraazacyclotetradecane;
1,1'-[4,4'-(2,2'-bipyridine)-bis-(methylene)]-bis-1,4,8,11-tetraazacyclotetradecane;
1,1'-[2,9-(1,10-phenanthroline)-bis-(methylene)]-bis-1,4,8,11-tetraazacyclotetradecane;
1,1'-[1,3-phenylene-bis-(methylene)]-bis-1,4,7,10-tetraazacyclotetradecane;
1,1'-[1,4-phenylene-bis-(methylene)]-bis-1,4,7,10-tetraazacyclotetradecane;
1,1'-[5-nitro-1,3-phenylenebis(methylene)]bis-1,4,8,11-tetraazacyclotetradecane;
1,1'-[2,4,5,6-tetrachloro-1,3-phenyleneis(methylene)]bis-1,4,8,11-tetraazacyclotetradecane;
1,1'-[2,3,5,6-tetrafluoro-1,4-phenylenebis(methylene)]bis-1,4,8,11-tetraazacyclotetradecane;
1,1'-[1,4-naphthylene-bis-(methylene)]bis-1,4,8,11-tetraazacyclotetradecane;
1,1'-[1,3-phenylenebis-(methylene)]bis-1,5,9-triazacyclododecane;
1,1'-[1,4-phenylene-bis-(methylene)]-1,5,9-triazacyclododecane;
1,1'-[2,5-dimethyl-1,4-phenylenebis-(methylene)]-bis-1,4,8,11-tetraazacyclotetradecane;
1,1'-[2,5-dichloro-1,4-phenylenebis-(methylene)]-bis-1,4,8,11-tetraazacyclotetradecane;
1,1'-[2-bromo-1,4-phenylenebis-(methylene)]-bis-1,4,8,11-tetraazacyclotetradecane;
1,1'-[6-phenyl-2,4-pyridinebis-(methylene)]-bis-1,4,8,11-tetraazacyclotetradecane;
7,7'-[1,4-phenylene-bis(methylene)]bis-3,7,11,17-tetraazabicyclo[13.3.1]heptadeca-1(17),13,15-triene;
7,7'-[1,4-phenylene-bis(methylene)]bis[15-chloro-3,7,11,17-tetraazabicyclo[13.3.1]heptadeca-1(17),13,15-triene];
7,7'-[1,4-phenylene-bis(methylene)]bis[15-methoxy-3,7,11,17-tetraazabicyclo[13.3.1]heptadeca-1(17),13,15-triene];
7,7'-[1,4-phenylene-bis(methylene)]bis-3,7,11,17-tetraazabicyclo[13.3.1]-heptadeca-13,16-triene-15-one;
7,7'-[1,4-phenylene-bis(methylene)]bis-4,7,10,17-tetraazabicyclo[13.3.1]-heptadeca-1(17),13,15-triene;
8,8'-[1,4-phenylene-bis(methylene)]bis-4,8,12,19-tetraazabicyclo[15.3.1]nonadeca-1(19),15,17-triene;
6,6'-[1,4-phenylene-bis(methylene)]bis-3,6,9,15-tetraazabicyclo[11.3.1]pentadeca-1(15),11,13-triene;
6,6'-[1,3-phenylene-bis(methylene)]bis-3,6,9,15-tetraazabicyclo[11.3.1]pentadeca-1(15),11,13-triene;
17,17'-[1,4-phenylene-bis(methylene)]bis-3,6,14,17,23,24-hexaazatricyclo[17.3.1.1^{8,12}]tetracosa-1(23),8,10,12(24),19,21-hexaene;
N-[1,4,8,11-Tetraazacyclotetradecanyl-1,4-phenylenebis(methylene)]-2-(amino-methyl)pyridine;
N-[1,4,8,11-Tetraazacyclotetradecanyl-1,4-phenylenebis(methylene)]-*N-*methyl-2-(aminomethyl)pyridine;
N-[1,4,8,11-Tetraazacyclotetradecanyl-1,4-phenylenebis(methylene)]-4-(aminomethyl)pyridine;
N-[1,4,8,11-Tetraazacyclotetradecanyl-1,4-phenylenebis(methylene)]-3-(aminomethyl)pyridine;
N-[1,4,8,11-Tetraazacyclotetradecanyl-1,4-phenylenebis(methylene)]-(2-aminomethyl-5-methyl)pyrazine;
N-[1,4,8,11-Tetraazacyclotetradecanyl-1,4-phenylenebis(methylene)]-2-(amino-ethyl)pyridine;
N-[1,4,8,11-Tetraazacyclotetradecanyl-1,4-phenylenebis(methylene)]-2-(aminomethyl)thiophene;
N-[1,4,8,11-Tetraazacyclotetradecanyl-1,4-phenylenebis(methylene)]-2-(amino-ethyl)mercaptan;
N-[1,4,8,11-Tetraazacyclotetradecanyl-1,4-phenylenebis(methylene)]-2-amino-benzylamine;
N-[1,4,8,11-Tetraazacyclotetradecanyl-1,4-phenylenebis(methylene)]-4-amino-benzylamine;
N-[1,4,8,11-Tetraazacyclotetradecanyl-1,4-phenylenebis(methylene)]-4-(amino-ethyl)imidazole;
N-[1,4,8,11-Tetraazacyclotetradecanyl-1,4-phenylenebis(methylene)]-benzylamine;
N-[1,4,8,11-Tetraazacyclotetradecanyl-1,4-phenylenebis(methylene)]-purine;
N-[1,4,8,11-Tetraazacyclotetradecanyl-1,4-phenylenebis(methylene)]-4-phenylpiperazine;
N-[4-(1,4,7-Triazacyclotetra-decanyl)-1,4-phenylenebis(methylene)]-2-(aminomethyl)pyridine;
N-[7-(4,7,10,17-Tetraazabicyclo[13.3.1]heptadeca-1(17),13,15-trienyl)-1,4-phenylenebis(methylene)]-2-(aminomethyl)pyridine;
N-[7-(4,7,10-Triazabicyclo[13.3.1]heptadeca-1(17),13,15-trienyl)-1,4-phenylenebis(methylene)]-2-(aminomethyl)pyridine;
N-[4-[4,7,10-Triazabicyclo[13.3.1]heptadeca-1(17),13,15-trienyl]-1,4-phenylenebis(methylene)]-2-(aminomethyl)pyridine;
N-[1-(1,4,7-Triazacyclotetra-decanyl)-1,4-phenylenebis(methylene)]-2-(aminomethyl)pyridine;
N-[4-[4,7,10,17-Tetraazabicyclo[13.3.1]heptadeca-1(17),13,15-trienyl]-1,4-phenylenebis(methylene)]-2-(aminomethyl)pyridine;
N-[3-(3,6,17-Triazabicyclo[13.3.1]heptadeca-1(17),13,15-trienyl)-1,4-phenylenebis(methylene)]-2-(aminomethyl)pyridine;
N-[3-(3,6,17-Triazabicyclo[13.3.1]heptadeca-1(17),13,15-trienyl)-1,3-phenylenebis(methylene)]-2-(aminomethyl)pyridine;
N-[4-(4,7,17-Triazabicyclo[13.3.1]heptadeca-1(17),13,15-trienyl)-1,4-phenylenebis(methylene)]-2-(aminomethyl)pyridine;
N-[7-(4,7,17-Triazabicyclo[13.3.1]heptadeca-1(17),13,15-trienyl)-1,4-phenylenebis(methylene)]-2-(aminomethyl)pyridine;
N-[6-(3,6,9-Triazabicyclo[11.3.1]pentadeca-1(15),11, 13-trienyl)-1,3-phenylenebis(methylene)]-2-(aminomethyl)pyridine;
N-[7-(4,10,17-Triazabicyclo[13.3.1]heptadeca-1(17),13,15-trienyl)-1,4-phenylenebis(methylene)]-2-(aminomethyl)pyridine;
N-[4-(1,7-Diazacyclotetradecanyl)-1,4-phenylenebis(methylene)]-2-(aminomethyl)pyridine;
N-[7-(4,10-Diazabicyclo[13.3.1]heptadeca-1(17),13,15-trienyl)-1,4-phenylenebis(methylene)]-2-(aminomethyl)pyridine;
N-[4-(11-Fluoro-1,4,7-triazacyclotetradecanyl)-1,4-phenylenebis(methylene)]-2-(aminomethyl)pyridine;
N-[4-(11,11-difluoro-1,4,7-triazacyclotetradecanyl)-1,4-phenylenebis(methylene)]-2-(aminomethyl)pyridine;
N-[4-(1,4,7-triazacyclotetradecan-2-one)-yl))-1,4-phenylenebis(methylene)]-2-(aminomethyl)pyridine;
N-[12-(5-oxa-1,9-diazacyclotetradecanyl)-1,4-phenylenebis(methylene)]-2-(aminomethyl)pyridine;
N-[4-(11-oxa-1,7-diazacyclotetradecanyl)-1,4-phenylenebis(methylene)]-2-(aminomethyl)pyridine;
N-[4-(11-thia-1,7-diazacyclotetradecanyl)-1,4-phenylenebis(methylene)]-2-(aminomethyl)pyridine;
N-[4-(11-sulfoxo-1,7-diazacyclotetradecanyl)-1,4-phenylenebis(methylene)]-2-(aminomethyl)pyridine;
N-[4-(11-sulfono-1,7-diazacyclotetradecanyl)-1,4-phenylenebis(methylene)]-2-(aminomethyl)pyridine;
N-[4-(1,4,7-triazacyclotetradecan-3-one)-yl))-1,4-phenylenebis(methylene)]-2-(aminomethyl)pyridine;
N-(2-pyridinylmethyl)-N'-(6,7,8,9-tetrahydro-5*H*-cyclohepta[*b*]pyridin-9-yl)-1,4-benzenedimethanamine;
N-(2-pyridinylmethyl)-N'-(5,6,7,8-tetrahydro-8-quinolinyl)-1,4-benzenedimethanamine;
N-(2-pyridinylmethyl)-N'-(6,7-dihydro-5*H*-cyclopenta[*b*]pyridin-7-yl)-1,4-benzenedimethanamine;
N-(2-pyridinylmethyl)-N'-(1,2,3,4-tetrahydro-1-naphthalenyl)-1,4-benzenedimethanamine;
N-(2-pyridinylmethyl)-N'-(1-naphthalenyl)-1,4-benzenedimethanamine;
N-(2-pyridinylmethyl)-N'-(8-quinolinyl)-1,4-benzenedimethanamine;
N-(2-pyridinylmethyl)-N'-[2-[(2-pyridinylmethyl)amino]ethyl]-N'-(1-methyl-1,2,3,4-tetrahydro-8-quinolinyl)-1,4-benzene dimethanamine;
N-(2-pyridinylmethyl)-N'-[2-[(1*H-*imidazol-2-ylmethyl)amino]ethyl]-N'-(1-methyl-1,2,3,4-tetrahydro-8-quinolinyl)-1,4-benzene dimethanamine;
N-(2-pyridinylmethyl)-N'-(1,2,3,4-tetrahydro-8-quinolinyl)-1,4-benzenedimethanamine;
N-(2-pyridinylmethyl)-N-[2-[(1*H*-imidazol-2-yhnethyl)amino]ethyl]-N'-(1,2,3,4-tetrahydro-1-naphthalenyl)-1,4-benzene dimethanamine;
N-(2-pyridinylmethyl)-N'-(2-phenyl-5,6,7,8-tetrahydro-8-quinolinyl)-1,4-benzenedimethanamine;
N,N'-bis(2-pyridinylmethyl)-N'-(2-phenyl-5,6,7,8-tetrahydro-8-quinolinyl)-1,4-benzenedimethanamine;
N-(2-pyridinylmethyl)-N'-(5,6,7,8-tetrahydro-5-quinolinyl)-1,4-benzenedimethanamine;
N-(2-pyridinylmethyl)-N'-(1*H*-imidazol-2-ylmethyl)-N'-(5,6,7,8-tetrahydro-5-quinolinyl)-1,4-benzenedimethanamine;
N-(2-pyridinylmethyl)-N'-(1*H-*imidazol-2-ylmethyl)-N'-(5,6,7,8-tetrahydro-8-quinolinyl)-1,4-benzenedimethanamine;
N-(2-pyridinylmethyl)-N'-[(2-amino-3-phenyl)propyl]-N'-(5,6,7,8-tetrahydro-8-quinolinyl)-1,4-benzenedimethanamine;
N-(2-pyridinylmethyl)-N'-(1*H-*imidazol-4-ylmethyl)-N'-(5,6,7,8-tetrahydro-8-quinolinyl)-1,4-benzenedimethanamine;
N-(2-pyridinylmethyl)-N'-(2-quinolinylmethyl)-N'-(5,6,7,8-tetrahydro-8-quinolinyl)-1,4-benzenedimethanamine;
N-(2-pyridinylmethyl)-N'-(2-(2-naphthoyl)aminoethyl)-N'-(5,6,7,8-tetrahydro-8-quinolinyl)-1,4-benzenedimethanamine;
N-(2-pyridinylmethyl)-N'-[(*S*)-(2-acetylamino-3-phenyl)propyl]-N'-(5,6,7,8-tetrahydro-8-quinolinyl)-1,4-benzenedimethanamine;
N-(2-pyridinylmethyl)-N'-[(*S*)-(2-acetylamino-3-phenyl)propyl]-N'-(5,6,7,8-tetrahydro-8-quinolinyl)-1,4-benzenedimethanamine;
N-(2-pyridinylmethyl)-N'-[3-((2-naphthalenylmethyl)amino)propyl]-N'-(5,6,7,8-tetrahydro-8-quinolinyl)-1,4-benzenedimethanamine;
N-(2-pyridinylmethyl)-N'-[2-(*S*)-pyrollidinylmethyl]-N'-(5,6,7,8-tetrahydro-8-quinolinyl)-1,4-benzenedimethanamine;
N-(2-pyridinylmethyl)-N'-[2-(*R*)-pyrollidinylmethyl]-N'-(5,6,7,8-tetrahydro-8-quinolinyl)-1,4-benzenedimethanamine;
N-(2-pyridinylmethyl)-N'-(3-pyrazolylmethyl]-N'-(5,6,7,8-tetrahydro-8-quinolinyl)-1,4-benzenedimethanamine;
N-(2-pyridinylmethyl)-N'-[2-pyrrolylmethyl]-N'-(5,6,7,8-tetrahydro-8-quinolinyl)-1,4-benzenedimethanamine;
N-(2-pyridinylmethyl)-N'-[2-thiopheneylmethyl]-N'-(5,6,7,8-tetrahydro-8-quinolinyl)-1,4-benzenedimethanamine
N-(2-pyridinylmethyl)-N'-[2-thiazolylmethyl]-N'-(5,6,7,8-tetrahydro-8-quinolinyl)-1,4-benzenedimethanamine;
N-(2-pyridinylmethyl)-N'-[2-furanylmethyl]-N'-(5,6,7,8-tetrahydro-8-quinolinyl)-1,4-benzenedimethanamine;
N-(2-pyridinylmethyl)-N'-[2-[(phenylmethyl)amino]ethyl]-N'-(5,6,7,8-tetrahydro-8-quinolinyl)-1,4-benzenedimethanamine;
N-(2-pyridinylmethyl)-N'-(2-aminoethyl)-N'-(5,6,7,8-tetrahydro-8-quinolinyl)-1,4-benzenedimethanamine;
N-(2-pyridinyhnethyl)-N'-3-pyrrolidinyl-N'-(5,6,7,8-tetrahydro-8-quinolinyl)-1,4-benzenedimethanamine
N-(2-pyridinylmethyl)-N'-4-piperidinyl-N'-(5,6,7,8-tetrahydro-8-quinolinyl)-1,4-benzenedimethanamine;
N-(2-pyridinylmethyl)-N'-[2-[(phenyl)amino]ethyl]-N'-(5,6,7,8-tetrahydro-8-quinolinyl)-1,4-benzenedimethanamine;
N-(2-pyridinylmethyl)-N'-(7-methoxy-1,2,3,4-tetrahydro-2-naphthalenyl)-1,4-benzenedimethanamine;
N-(2-pyridinylmethyl)-N'-(6-methoxy-1,2,3,4-tetrahydro-2-naphthalenyl)-1,4-benzenedimethanamine;
N-(2-pyridinylmethyl)-N'-(1-methyl-1,2,3,4-tetrahydro-2-naphthalenyl)-1,4-benzenedimethanamine;
N-(2-pyridinylmethyl)-N'-(7-methoxy-3,4-dihydronaphthalenyl)-1-(aminomethyl)-4-benzamide;
N-(2-pyridinylmethyl)-N'-(6-methoxy-3,4-dihydronaphthalenyl)-1-(aminomethyl)-4-benzamide;
N-(2-pyridinylmethyl)-N'-(1*H-*imidazol-2-ylmethyl)-N'-(7-methoxy-1,2,3,4-tetrahydro-2-naphthalenyl)-1,4-benzenedimethanamine;
N-(2-pyridinylmethyl)-N'-(8-hydroxy-1,2,3,4-tetrahydro-2-naphthalenyl)-1,4-benzenedimethanamine;
N-(2-pyridinylmethyl)-N'-(1*H*-imidazol-2-ylmethyl)-N'-(8-hydroxy-1,2,3,4-tetrahydro-2-naphthalenyl)-1,4-benzenedimethanamine;
N-(2-pyridinylmethyl)-N'-(8-Fluoro-1,2,3,4-tetrahydro-2-naphthalenyl)-1,4-benzenedimethanamine;
N-(2-pyridinylmethyl)-N'-(1*H*-imidazol-2-ylmethyl)-N'-(8-Fluoro-1,2,3,4-tetrahydro-2-naphthalenyl)-1,4-benzenedimethanamine;
N-(2-pyridinylmethyl)-N'-(5,6,7,8-tetrahydro-7-quinolinyl)-1,4-benzenedimethanamine;
N-(2-pyridinylmethyl)-N'-(1H-imidazol-2-ylmethyl)-N'-(5,6,7,8-tetrahydro-7-quinolinyl)-1,4-benzenedimethanamine;
N-(2-pyridinylmethyl)-N'-[2-[(2-naphthalenylmethyl) amino]ethyl]-N'-(5,6,7,8-tetrahydro-8-quinolinyl)-1,4-benzenedimethanamine;
N-(2-pyridinylmethyl)-N'-[2-(isobutylamino)ethyl]-N'-(5,6,7,8-tetrahydro-8-quinolinyl)-1,4-benzenedimethanamine;
N-(2-pyridinylmethyl)-N'-[2-[(2-pyridinylmethyl) amino]ethyl]-N'-(5,6,7,8-tetrahydro-8-quinolinyl)-1,4-benzenedimethanamine;
N-(2-pyridinylmethyl)-N'-[2-[(2-furanylmethyl)amino]ethyl]-N'-(5,6,7,8-tetrahydro-8-quinolinyl)-1,4-benzenedimethanamine;
N-(2-pyridinylmethyl)-N'-(2-guanidinoethyl)-N'-(5,6,7,8-tetrahydro-8-quinolinyl)-1,4-benzenedimethanamine;
N-(2-pyridinylmethyl)-N'-[2-[bis-[(2-methoxy)phenylmethyl]amino]ethyl]-N'-(5,6,7,8-tetrahydro-8-quinolinyl)-1,4-benzene dimethanamine;
N-(2-pyridinylmethyl)-N'-[2-[(1*H*-imidazol-4-ylmethyl)amino]ethyl]-N'-(5,6,7,8-tetrahydro-8-quinolinyl)-1,4-benzene dimethanamine;
N-(2-pyridinylmethyl)-N'-[2-[(1*H*-imidazol-2-ylmethyl)amino]ethyl]-N'-(5,6,7,8-tetrahydro-8-quinolinyl)-1,4-benzenedimethanamine;
N-(2-pyridinylmethyl)-N'-[2-(phenylureido)ethyl]-N'-(5,6,7,8-tetrahydro-8-quinolinyl)-1,4-benzenedimethanamine;
N-(2-pyridinylmethyl)-N'-[[N"-(n-butyl)carboxamido]methyl] -N'-(5,6,7,8-tetrahydro-8-quinolinyl)-1,4-benzenedimethanamine;
N-(2-pyridinylmethyl)-N'-(carboxamidomethyl)-N'-(5,6,7,8-tetrahydro-8-quinolinyl)-1,4-benzenedimethanamine;
N-(2-pyridinylmethyl)-N'-[(N"-phenyl)carboxamidomethyl]-N'-(5,6,7,8-tetrahydro-8-quinolinyl)-1,4-benzenedimethanamine;
N-(2-pyridinylmethyl)-N'-(carboxymethyl)-N'-(5,6,7,8-tetrahydro-8-quinolinyl)-1,4-benzenedimethanamine;
N-(2-pyridinylmethyl)-N'-(phenylmethyl)-N'-(5,6,7,8-tetrahydro-8-quinolinyl)-1,4-benzenedimethanamine;
N-(2-pyridinylmethyl)-N'-(1*H*-benzimidazol-2-ylmethyl)-N'-(5,6,7,8-tetrahydro-8-quinolinyl)-1,4-benzenedimethanamine;
N-(2-pyridinylmethyl)-N'-(5,6-dimethyl-1*H*-benzimidazol-2-ylmethyl)-N'-(5,6,7,8-tetrahydro-8-quinolinyl)-1,4-benzenedimethanamine (hydrobromide salt);
N-(2-pyridinylmethyl)-N'-(5-nitro-1*H*-benzimidazol-2-ylmethyl)-N'-(5,6,7,8-tetrahydro-8-quinolinyl)-1,4-benzenedimethanamine;
N-(2-pyridinylmethyl)-N'-[(1*H*)-5-azabenzimidazol-2-ylmethyl]-N'-(5,6,7,8-tetrahydro-8-quinolinyl)-1,4-benzenedimethanamine;
N-(2-pyridinylmethyl)-N-(4-phenyl-1*H* imidazol-2-ylmethyl)-N'-(5,6,7,8-tetrahydro-8-quinolinyl)-1,4-benzenedimethanamine;
N-(2-pyridinylmethyl)-N'-[2-(2-pyridinyl)ethyl]-N'-(5,6,7,8-tetrahydro-8-quinolinyl)-1,4-benzenedimethanamine;
N-(2-pyridinylmethyl)-N'-(2-benzoxazolyl)-N'-(5,6,7,8-tetrahydro-8-quinolinyl)-1,4-benzenedimethanamine;
N-(2-pyridinylmethyl)-N'-(*trans*-2-aminocyclohexyl)-N'-(5,6,7,8-tetrahydro-8-quinolinyl)-1,4-benzenedimethanamine;

N-(2-pyridinylmethyl)-N'-(2-phenylethyl)-N'-(5,6,7,8-tetrahydro-8-quinolinyl)-1,4-benzenedimethanamine;
N-(2-pyridinylmethyl)-N'-(3-phenylpropyl)-N'-(5,6,7,8-tetrahydro-8-quinolinyl)-1,4-benzenedimethanamine;
N-(2-pyridinylmethyl)-N'-(*trans*-2-aminocyclopentyl)-N'-(5,6,7,8-tetrahydro-8-quinolinyl)-1,4-benzenedimethanamine;
N-[[4-[[(2-pyridinylmethyl)amino]methyl]phenyl]methyl]-N-(5,6,7,8-tetrahydro-8-quinolinyl)-glycinamide;
N-[[4-[[(2-pyridinylmethyl)amino]methyl]phenyl]methyl]-N-(5,6,7,8-tetrahydro-8-quinolinyl)-(L)-alaninamide;
N-[[4-[[(2-pyridinylmethyl)amino]methyl]phenyl]methyl]-N-(5,6,7,8-tetrahydro-8-quinolinyl)-(L)-aspartamide;
N-[[4-[[(2-pyridinylmethyl)amino]methyl]phenyl]methyl]-N-(5,6,7,8-tetrahydro-8-quinolinyl)-pyrazinamide;
N-[[4-[[(2-pyridinylmethyl)amino]methyl]phenyl]methyl]-N-(5,6,7,8-tetrahydro-8-quinolinyl)-(L)-prolinamide;
N-[[4-[[(2-pyridinylmethyl)amino]methyl]phenyl]methyl]-N-(5,6,7,8-tetrahydro-8-quinolinyl)-(L)-lysinamide;
N-[[4-[[(2-pyridinylmethyl)amino]methyl]phenyl]methyl]-N-(5,6,7,8-tetrahydro-8-quinolinyl)-benzamide;
N-[[4-[[(2-pyridinylmethyl)amino]methyl]phenyl]methyl]-N-(5,6,7,8-tetrahydro-8-quinolinyl)-picolinamide;
N'-Benzyl-N-[[4-[[(2-pyridinylmethyl) amino]methyl]phenyl]methyl]-N-(5,6,7,8-tetrahydro-8-quinolinyl)-urea;
N'-phenyl-N-[[4-[[(2-pyridinylmethyl) amino]methyl]phenyl]methyl]-N-(5,6,7,8-tetrahydro-8-quinolinyl)-urea;
N-(6,7,8,9-tetrahydro-5*H-*cyclohepta[*bacteria*pyridin-9-yl)-4-[[(2-pyridinylmethyl)amino]methyl]benzamide;
N-(5,6,7,8-tetrahydro-8-quinolinyl)-4-[[(2-pyridinylmethyl)amino]methyl]benzamide;
N,N'-bis(2-pyridinylmethyl)-N'-(5,6,7,8-tetrahydro-8-quinolinyl)-1,4-benzenedimethanamine;
N,N'-bis(2-pyridinylmethyl)-N'-(6,7,8,9-tetrahydro-5*H*-cyclohepta[*bacteria*pyridin-9-yl)-1,4-benzenedimethanamine;
N,N'-bis(2-pyridinylmethyl)-N'-(6,7-dihydro-5*H-*cyclopenta[*bacteria*pyridin-7-yl)-1,4-benzenedimethanamine;
N,N'-bis(2-pyridinylmethyl)-N'-(1,2,3,4-tetrahydro-1-naphthalenyl)-1,4-benzenedimethanamine;
N,N'-bis(2-pyridinylmethyl)-N'-[(5,6,7,8-tetrahydro-8-quinolinyl)methyl]-1,4-benzenedimethanamine;
N,N'-bis(2-pyridinylmethyl)-N'[(6,7-dihydro-5*H*-cyclopenta[*bacteria*pyridin-7-yl)methyl]-1,4-benzenedimethanamine;
N-(2-pyridinylmethyl)-N-(2-methoxyethyl)-N'-(5,6,7,8-tetrahydro-8-quinolinyl)-1,4-benzenedimethanamine;
N-(2-pyridinylmethyl)-N-[2-(4-methoxyphenyl)ethyl]-N'-(5,6,7,8-tetrahydro-8-quinolinyl)-1,4-benzenedimethanamine;
N,N'-bis(2-pyridinylmethyl)-1,4-(5,6,7,8-tetrahydro-8-quinolinyl)benzenedimethanamine;
N-[(2,3-dimethoxyphenyl)methyl]-N'-(2-pyridinylmethyl)-N-(5,6,7,8-tetrahydro-8-quinolinyl)-1,4-benzenedimethanamine;
N,N'-bis(2-pyridinylinethyl)-N-[1-(N"-phenyl-N"-methylureido)-4-piperidinyl]-1,3-benzenedimethanamine;
N,N'-bis(2-pyridinylmethyl)-N-[N"-p-toluenesulfonylphenylalanyl)-4-piperidinyl]-1,3-benzenedimethanamine;
N,N'-bis(2-pyridinylmethyl)-N-[1-[3-(2-chlorophenyl)-5-methyl-isoxazol-4-oyl]-4-piperidinyl]-1,3-benzenedimethanamine;
N-[(2-hydroxyphenyl)methyl]-N'-(2-pyridinylmethyl)-N-(6,7,8,9-tetrahydro-5H-cyclohepta[bacteriapyridin-9-yl)-1,4-benzenedimethanamine;
N-[(4-cyanophenyl)methyl]-N'-(2-pyridinylmethyl)-N-(6,7,8,9-tetrahydro-5H-cyclohepta[bacteriapyridin-9-yl)-1,4-benzenedimethanamine;
N-[(4-cyanophenyl)methyl]-N'-(2-pyridinylmethyl)-N-(5,6,7,8-tetrahydro-8-quinolinyl)-1,4-benzenedimethanamine;
N-[(4-acetamidophenyl)methyl]-N'-(2-pyridinylmethyl)-N-(5,6,7,8-tetrahydro-8-quinolinyl)-1,4-benzenedimethanamine;
N-[(4-phenoxyphenyl)methyl]-N'-(2-pyridinylmethyl)-N-(6,7,8,9-tetrahydro-5H-cyclohepta[bacteriapyridin-9-yl)-1,4-benzenedimethanamine;
N-[(1-methyl-2-carboxamido)ethyl]-N,N'-bis(2-pyridinylmethyl)-1,3-benzenedimethanamine;
N-[(4-benzyloxyphenyl)methyl]-N'-(2-pyridinylmethyl)-N-(6,7,8,9-tetrahydro-5H-cyclohepta[bacteriapyridin-9-yl)-1,4-benzenedimethanamine;
N-[(thiophene-2-yl)methyl]-N'-(2-pyridinylmethyl)-N-(6,7,8,9-tetrahydro-5H-cyclohepta[bacteriapyridin-9-yl)-1,4-benzenedimethanamine;
N-[1-(benzyl)-3-pyrrolidinyl]-N,N'-bis(2-pyridinylmethyl)-1,3-benzenedimethanamine;
N-[[1-methyl-3-(pyrazol-3-yl)]propyl]-N,N'-bis(2-pyridinylmethyl)-1,3-benzenedimethanamine;
N-[1-(phenyl)ethyl]-N,N'-bis(2-pyridinylmethyl)-1,3-benzenedimethanamine;
N-[(3,4-methylenedioxyphenyl)methyl]-N'-(2-pyridinylmethyl)-N-(6,7,8,9-tetrahydro-5H-cyclohepta[b]pyridin-9-yl)-1,4-benzenedimethanamine;
N-[1-benzyl-3-carboxymethyl-4-piperidinyl]-N,N'-bis(2-pyridinylmethyl)-1,3-benzenedimethanamine;
N-[(3,4-methylenedioxyphenyl)methyl]-N'-(2-pyridinylmethyl)-N-(5,6,7,8-tetrahydro-8-quinolinyl)-1,4-benzenedimethanamine;
N-(3-pyridinylmethyl)-N'-(2-pyridinylmethyl)-N-(6,7,8,9-tetrahydro-5H-cyclohepta[b]pyridin-9-yl)-1,4-benzenedimethanamine;
N-[[1-methyl-2-(2-tolyl)carboxamido]ethyl]-N,N'-bis(2-pyridinylmethyl)-1,3-benzenedimethanamine;
N-[(1,5-dimethyl-2-phenyl-3-pyrazolinone-4-yl)methyl]-N'-(2-pyridinylmethyl)-N-(5,6,7,8-tetrahydro-8-quinolinyl)-1,4-benzenedimethanamine;
N-[(4-propoxyphenyl)methyl]-N'-(2-pyridinylmethyl)-N-(6,7,8,9-tetrahydro-5H-cyclohepta[b]pyridin-9-yl)-1,4-benzenedimethanamine;
N-(1-phenyl-3,5-dimethylpyrazolin-4-ylmethyl)-N'-(2-pyridinylmethyl)-N-(5,6,7,8-tetrahydro-8-quinolinyl)-1,4-benzenedimethanamine;
N-[1*H*-imidazol-4-ylnlethyl]-N,N'-bis(2-pyridinylmethyl)-1,3-benzenedimethanamine;
N-[(3-methoxy-4,5-methylenedioxyphenyl)methyl]-N'-(2-pyridinylmethyl)-N-(6,7,8,9-tetrahydro-5H-cyclohepta[b]pyridin-9-yl)-1,4-benzenedimethanamine;
N-[(3-cyanophenyl)methyl]-N'-(2-pyridinylmethyl)-N-(6,7,8,9-tetrahydro-5H-cyclohepta[b]pyridin-9-yl)-1,4-benzenedimethanamine;
N-[(3-cyanophenyl)methyl]-N'-(2-pyridinylmethyl)-N-(5,6,7,8-tetrahydro-8-quinolinyl)-1,4-benzenedimethanamine;
N-(5-ethylthiophene-2-ylmethyl)-N'-(2-pyridinylmetliyl)-N-(6,7,8,9-tetrahydro-5H-cyclohepta[b]pyridin-9-yl)-1,4-benzenedimethanamine;
N-(5-ethylthiophene-2-ylmethyl)-N'-(2-pyridinylmethyl)-N-(5,6,7,8-tetrahydro-8-quinolinyl)-1,4-benzenedimethanamine;
N-[(2,6-difluorophenyl)methyl]-N'-(2-pyridinylmethyl)-N-(6,7,8,9-tetrahydro-5H-cyclohepta[b]pyridin-9-yl)-1,4-benzenedimethanamine;
N-[(2,6-difluorophenyl)methyl]-N'-(2-pyridinylmethyl)-N-(5,6,7,8-tetrahydro-8-quinolinyl)-1,4-benzenedimethanamine;
N-[(2-difluoromethoxyphenyl)methyl]-N'-(2-pyridinylmethyl)-N-(6,7,8,9-tetrahydro-5H-cyclohepta[b]pyridin-9-yl)-1,4-benzenedimethanamine;
N-(2-difluoromethoxyphenylmethyl)-N'-(2-pyridinylmethyl)-N-(5,6,7,8-tetrahydro-8-quinolinyl)-1,4-benzenedimethanamine;
N-(1,4-benzodioxan-6-ylmethyl)-N'-(2-pyridinylmethyl)-N-(6,7,8,9-tetrahydro-5H-cyclohepta[b]pyridin-9-yl)-1,4-benzenedimethanamine;
N, N'-bis(2-pyridinylmethyl)-N-[1-(N"-phenyl-N"-methylureido)-4-piperidinyl]-1,4-benzenedimethanamine;
N, N'-bis(2-pyridinylmethyl)-N-[N"-p-toluenesulfonylphenylalanyl)-4-piperidinyl]-1,4-benzenedimethanamine;
N-[1-(3-pyridinecarboxamido)-4-piperidinyl]-N,N'-bis(2-pyridinylmethyl)-1,4-benzenedimethanamine;
N-[1-(cyclopropylcarboxamido)-4-piperidinyl]-N,N'-bis(2-pyridinylmethyl)-1,4-benzenedimethanamine;
N-[1-(1-phenylcyclopropylcarboxamido)-4-piperidinyl]-N,N'-bis(2-pyridinylmethyl)-1,4-benzenedimethanamine;
N-(1,4-benzodioxan-6-ylmethyl)-N'-(2-pyridinylmethyl)-N-(5,6,7,8-tetrahydro-8-quinolinyl)-1,4-benzenedimethanamine;
N-[1-[3-(2-chlorophenyl)-5-methyl-isoxazol-4-carboxamido]-4-piperidinyl]-N,N'-bis(2-pyridinylmethyl)-1,4-benzenedimethanamine;
N-[1-(2-thiomethylpyridine-3-carboxamido)-4-piperidinyl]-N,N'-bis(2-pyridinylmethyl)-1,4-benzenedimethanamine;
N-[(2,4-difluorophenyl)methyl]-N'-(2-pyridinylmethyl)-N-(5,6,7,8-tetrahydro-8-quinolinyl)-1,4-benzenedimethanamine;
N-(1-methylpyrrol-2-ylmethyl)-N'-(2-pyridinylmethyl)-N-(5,6,7,8-tetrahydro-8-quinolinyl)-1,4-benzenedimethanamine;
N-[(2-hydroxyphenyl)methyl]-N'-(2-pyridinylmethyl)-N-(5,6,7,8-tetrahydro-8-quinolinyl)-1,4-benzenedimethanamine;
N-[(3-methoxy-4,5-methylenedioxyphenyl)methyl]-N'-(2-pyridinylmethyl)-N-(5,6,7,8-tetrahydro-8-quinolinyl)-1,4-benzenedimethanamine;
N-(3-pyridinylmethyl)-N'-(2-pyridinylmethyl)-N-(5,6,7,8-tetrahydro-8-quinolinyl)-1,4-benzenedimethanamine;
N-[2-(N"-morpholinomethyl)-1-cyclopentyl]-N,N'-bis(2-pyridinylmethyl)-1,4-benzenedimethanamine;
N-[(1-methyl-3-piperidinyl)propyl]-N,N'-bis(2-pyridinylmethyl)-1,4-benzenedimethanamine;
N-(1-methylbenzimidazol-2-ylmethyl)-N'-(2-pyridinylmethyl)-N-(5,6,7,8-tetrahydro-8-quinolinyl)-1,4-benzenedimethanamine;
N-[1-(benzyl)-3-pyrrolidinyl]-N,N'-bis(2-pyridinylmethyl)-1,4-benzenedimethanamine;
N-[[(1-phenyl-3-(N"-morpholino)]propyl]-N,N'-bis(2-pyridinylmethyl)-1,4-benzenedimethanamine;
N-[1-(iso-propyl)-4-piperidinyl]-N,N'-bis(2-pyridinylmethyl)-1,4-benzenedimethanamine;
N-[1-(ethoxycarbonyl)-4-piperidinyl]-N'-(2-pyridinylmethyl)-N-(5,6,7,8-tetrahydro-8-quinolinyl)-1,4-benzenedimethanamine;
N-[(1-methyl-3-pyrazolyl)propyl]-N'-(2-pyridinylmethyl)-N-(5,6,7,8-tetrahydro-8-quinolinyl)-1,4-benzenedimethanamine;
N-[1-methyl-2-(N",N"-diethylcarboxamido)ethyl]-N,N'-bis(2-pyridinylmethyl)-1,4-benzenedimethanamine;
N-[(1-methyl-2-phenylsulfonyl)ethyl]-N'-(2-pyridinylmethyl)-N-(5,6,7,8-tetrahydro-8-quinolinyl)-1,4-benzenedimethanamine;
N-[(2-chloro-4,5-methylenedioxyphenyl)methyl]-N'-(2-pyridinylmethyl)-N-(5,6,7,8-tetrahydro-8-quinolinyl)-1,4-benzenedimethanamine;
N-[1-methyl-2-[N"-(4-chlorophenyl)carboxamido]ethyl]-N'-(2-pyridinylmethyl)-N-(5,6,7,8-tetrahydro-8-quinolinyl)-1,4-benzenedimethanamine;
N-(1-acetoxyindol-3-ylmethyl)-N'-(2-pyridinylmethyl)-N-(6,7,8,9-tetrahydro-5H-cyclohepta[b]pyridin-9-yl)-1,4-benzenedimethanamine;
N-[(3-benzyloxy-4-methoxyphenyl)methyl]-N'-(2-pyridinylmethyl)-N-(6,7,8,9-tetrahydro-5H-cyclohepta[b]pyridin-9-yl)-1,4-benzenedimethanamine;
N-(3-quinolylmethyl)-N'-(2-pyridinylmethyl)-N-(5,6,7,8-tetrahydro-8-quinolinyl)-1,4-benzenedimethanamine;
N-[(8-hydroxy)-2-quinolylmethyl]-N'-(2-pyridinylmethyl)-N-(6,7,8,9-tetrahydro-5H-cyclohepta[b]pyridin-9-yl)-1,4-benzenedimethanamine;
N-(2-quinolylmethyl)-N'-(2-pyridinylmethyl)-N-(6,7,8,9-tetrahydro-5H-cyclohepta[b]pyridin-9-yl)-1,4-benzenedimethanamine;
N-[(4-acetamidophenyl)methyl]-N'-(2-pyridinylmethyl)-N-(6,7,8,9-tetrahydro-5H-cyclohepta[b]pyridin-9-yl)-1,4-benzenedimethanamine;
N-[1H-imidazol-2-ylmethyl]-N,N'-bis(2-pyridinylmethyl)-1,4-benzenedimethanamine;
N-(3-quinolylmethyl)-N'-(2-pyridinylmethyl)-N-(6,7,8,9-tetrahydro-5H-cyclohepta[b]pyridin-9-yl)-1,4-benzenedimethanamine;
N-(2-thiazolylmethyl)-N'-(2-pyridinylmethyl)-N-(6,7,8,9-tetrahydro-5H-cyclohepta[b]pyridin-9-yl)-1,4-benzenedimethanamine;
N-(4-pyridinylmethyl)-N'-(2-pyridinylmethyl)-N-(6,7,8,9-tetrahydro-5H-cyclohepta[b]pyridin-9-yl)-1,4-benzenedimethanamine;
N-[(5-benzyloxy)benzo[b]pyrrol-3-ylmethyl]-N,N'-bis(2-pyridinylmethyl)-1,4-benzenedimethanamine;
N-(1-methylpyrazol-2-ylmethyl)-N'-(2-pyridinylmethyl)-N-(6,7,8,9-tetrahydro-5H-cyclohepta[b]pyridin-9-yl)-1,4-benzenedimethanamine;
N-[(4-methyl)-1H-imidazol-5-ylmethyl]-N,N'-bis(2-pyridinylmethyl)-1,4-benzenedimethanamine;
N-[[(4-dimethylamino)-1-napthalenyl]methyl]-N,N'-bis(2-pyridinylmethyl)-1,4-benzenedimethanamine;
N-[1,5-dimethyl-2-phenyl-3-pyrazolinone-4-ylmethyl]-N,N'-bis(2-pyridinylmethyl)-1,4-benzenedimethanamine;
N-[1-[(1-acetyl-2-(R)-prolinyl]-4-piperidinyl]-N-[2-(2-pyridinyl)ethyl]-N'-(2-pyridinylmethyl)-1,3-benzenedimethanamine;
N-[1-[2-acetamidobenzoyl-4-piperidinyl]-4-piperidinyl]-N-[2-(2-pyridinyl)ethyl]-N'-(2-pyridinylmethyl)-1,3-benzenedimethanamine;
N-[(2-cyano-2-phenyl)ethyl]-N'-(2-pyridinylmethyl)-N-(6,7,8,9-tetrahydro-5H-cyclohepta[b]pyridin-9-yl)-1,4-benzenedimethanamine;
N-[(N"-acetyltryptophanyl)-4-piperidinyl]-N-[2-(2-pyridinyl)ethyl]-N'-(2-pyridinylmethyl)-1,3-benzenedimethanamine;
N-[(N"-benzoylvalinyl)-4-piperidinyl]-N-[2-(2-pyridinyl)ethyl]-N'-(2-pyridinylmethyl)-1,3-benzenedimethanamine;
N-[(4-dimethylaminophenyl)methyl]-N'-(2-pyridinylmethyl)-N-(6,7,8,9-tetrahydro-5H-cyclohepta[b]pyridin-9-yl)-1,4-benzenedimethanamine;
N-(4-pyridinylmethyl)-N'-(2-pyridinylmethyl)-N-(5,6,7,8-tetrahydro-8-quinolinyl)-1,4-benzenedimethanamine;
N-(1-methylbenzimadazol-2-ylmethyl)-N'-(2-pyridinylmethyl)-N-(6,7,8,9-tetrahydro-5H-cyclohepta[b]pyridin-9-yl)-1,4-benzenedimethanamine;
N-[1-butyl-4-piperidinyl]-N-[2-(2-pyridinyl)ethyl]-N'-(2-pyridinylmethyl)-1,3-benzenedimethanamine;
N-[1-benzoyl-4-piperidinyl]-N-[2-(2-pyridinyl)ethyl]-N'-(2-pyridinylmethyl)-1,3-benzenedimethanamine;
N-[1-(benzyl)-3-pyrrolidinyl]-N-[2-(2-pyridinyl)ethyl]-N'-(2-pyridinylmethyl)-1,3-benzenedimethanamine;
N-[(1-methyl)benzo[b]pyrrol-3-ylmethyl]-N-[2-(2-pyridinyl)ethyl]-N'-(2-pyridinylmethyl)-1,3-benzenedimethanamine;
N-[1H-imidazol-4-ylmethyl]-N-[2-(2-pyridinyl)ethyl]-N'-(2-pyridinylmethyl)-1,3-benzenedimethanamine;
N-[1-(benzyl)-4-piperidinyl]-N-[2-(2-pyridinyl)ethyl]-N'-(2-pyridinylmethyl)-1,4-benzenedimethanamine;
N-[1-methylbenzimidazol-2-ylmethyl]-N-[2-(2-pyridinyl)ethyl]-N'-(2-pyridinylmethyl)-1,4-benzenedimethanamine;
N-[(2-phenyl)benzo[b]pyrrol-3-ylmethyl]-N-[2-(2-pyridinyl)ethyl]-N'-(2-pyridinylmethyl)-1,4-benzenedimethanamine;
N-[(6-methylpyridin-2-yl)methyl]-N'-(2-pyridinylmethyl)-N-(5,6,7,8-tetrahydro-8-quinolinyl)-1,4-benzenedimethanamine;
N-(3-methyl-1H-pyrazol-5-ylmethyl)-N'-(2-pyridinylmethyl)-N-(5,6,7,8-tetrahydro-8-quinolinyl)-1,3-benzenedimethanamine;
N-[(2-methoxyphenyl)methyl]-N'-(2-pyridinylmethyl)-N-(5,6,7,8-tetrahydro-8-quinolinyl)-1,3-benzenedimethanamine;
N-[(2-ethoxyphenyl)methyl]-N'-(2-pyridinylmethyl)-N-(6,7,8,9-tetrahydro-5H-cyclohepta[b]pyridin-9-yl)-1,3-benzenedimethanamine;
N-(benzyloxyethyl)-N'-(2-pyridinylmethyl)-N-(5,6,7,8-tetrahydro-8-quinolinyl)-1,3-benzenedimethanamine;
N-[(2-ethoxy-1-naphthalenyl)methyl]-N'-(2-pyridinylmethyl)-N-(5,6,7,8-tetrahydro-8-quinolinyl)-1,3-benzenedimethanamine;
N-[(6-methylpyridin-2-yl)methyl]-N'-(2-pyridinylmethyl)-N-(5,6,7,8-tetrahydro-8-quinolinyl)-1,3-benzenedimethanamine;
1-[[4-[[(2-pyridinylmethyl)amino]methyl] phenyl]methyl]guanidine;
N-(2-pyridinylmethyl)-N-(8-methyl-8-azabicyclo[3.2.1]octan-3-yl)-1,4-benzenedimethanamine;
1-[[4-[[(2-pyridinylmethyl)amino]methyl]phenyl]methyl]homopiperazine;
1-[[3-[[(2-pyridinylmethyl)amino]methyl]phenyl]methyl]homopiperazine;
*trans* and *cis*-1-[[4-[[(2-pyridinylmethyl)amino]methyl]phenyl]methyl]-3,5-piperidinediamine;
N,N'-[1,4-Phenylenebis(methylene)]bis-4-(2-pyrimidyl) piperazine;
1-[[4-[[(2-pyridinylmethyl)amino]methyl]phenyl]methyl]-1-(2-pyridinyl)methylamine;
2-((2-pyridinyl)-5-[[(2-pyridinylmethyl)amino]methyl]-1,2,3,4-tetrahydroisoquinoline;
1-[[4-[[(2-pyridinylmethyl)amino]methyl]phenyl]methyl]-3,4-diaminopyrrolidine;
1-[[4-[[(2-pyridinylmethyl)amino]methyl]phenyl]methyl]-3,4-diacetylaminopyrrolidine;
8-[[4-[[(2-pyridinylmethyl)amino]methyl]phenyl]methyl]-2,5,8-triaza-3-oxabicyclo[4.3.0]nonane;
8-[[4-[[(2-pyridinylmethyl)amino]methyl]phenyl]methyl]-2,5,8-triazabicyclo[4.3.0]nonane;
(4-Aminomethyl-pyridin-3-ylmethyl)-(1H-benzoimidazol-2-ylmethyl)-(5,6,7,8-tetrahydro-quinolin-8-yl)-amine;
(3-Aminomethyl-pyridin-4-ylmethyl)-(1H-benzoimidazol-2-ylmethyl)-(5,6,7,8-tetrahydro-quinolin-8-yl)-amine;
1-((3-Aminomethyl-4- {[(1H-benzoimidazol-2-ylmethyl)-(5,6,7,8-tetrahydro-quinolin-8-yl)-amino]-methyl}-phenyl)-ethanone;
1-((5-Aminomethyl-2-{[(1H-benzoimidazol-2-ylmethyl)-(5,6,7,8-tetrahydro-quinolin-8-yl)-amino]-methyl}-phenyl)-ethanone;
3-Aminomethyl-4- {[(1H-benzoimidazol-2-ylmethyl)-(5,6,7,8-tetrahydro-quinolin-8-yl)-amino]-methyl}-benzenesulfonamide;
5-Aminomethyl-2-{[(1H-benzoimidazol-2-ylmethyl)-(5,6,7,8-tetrahydro-quinolin-8-yl)-amino]-methyl}-benzenesulfonamide;
N-(3-Aminomethyl-4-{[(1H-benzoimidazol-2-ylmethyl)-(5,6,7,8-tetrahydro-quinolin-8-yl)-amino]-methyl}-benzyl)-hydroxylamine;
N-(5-Aminomethyl-2-{[(1H-benzoimidazol-2-ylmethyl)-(5,6,7,8-tetrahydro-quinolin-8-yl)-amino]-methyl}-benzyl)-hydroxylamine;
N-(3-Aminomethyl-4- {[(1H-benzoimidazol-2-ylmethyl)-(5,6,7,8-tetrahydro-quinolin-8-yl)-amino]-methyl}-benzyl)-O-methyl-hydroxylamine;
N-(5-Aminomethyl-2- {[(1H-benzoimidazol-2-ylmethyl)-(5,6,7,8-tetrahydro-quinolin-8-yl)-amino]-methyl}-benzyl)-O-methyl-hydroxylamine;
(4-Aminomethyl-2-methoxymethyl-benzyl)-(1H-benzoimidazol-2-ylmethyl)-(5,6,7,8-tetrahydro-quinolin-8-yl)-amine;
(2-Aminomethyl-4-methoxymethyl-benzyl)-(1H-benzoimidazol-2-ylmethyl)-(5,6,7,8-tetrahydro-quinolin-8-yl)-amine;
N-(2- {[(1H-Benzoimidazol-2-ylmethyl)-(5,6,7,8-tetrahydro-quinolin-8-yl)-amino]-methyl}-benzyl)-formamide;
N-(4- {[(1H-Benzoimidazol-2-ylmethyl)-(5,6,7,8-tetrahydro-quinolin-8-yl)-amino]-methyl}-benzyl)-formamide;
N-(2-{[(1H-Benzoimidazol-2-ylmethyl)-(5,6,7,8-tetrahydro-quinolin-8-yl)-amino]-methyl}-benzyl)-hydroxylamine;
(1H-Benzoimidazol-2-ylmethyl)-(2,6-bis-aminomethyl-benzyl)-(5,6,7,8-tetrahydro-quinolin-8-yl)-amine;
(3-Aminomethyl-2- {[(1H-benzoimidazol-2-ylmethyl)-(5,6,7,8-tetrahydro-quinolin-8-yl)-amino]-methyl}-phenyl)-methanol;
(2-Aminomethyl-6-methoxymethyl-benzyl)-(1H-benzoimidazol-2-ylmethyl)-(5,6,7,8-tetrahydro-quinolin-8-yl)-amine;
N-(3-Aminomethyl-2- {[(1H-benzoimidazol-2-ylmethyl)-(5,6,7,8-tetrahydro-quinolin-8-yl)-amino]-methyl}-benzyl)-hydroxylamine;
N-(3-Aminomethyl-2-{[(1H-benzoimidazol-2-ylmethyl)-(5,6,7,8-tetrahydro-quinolin-8-yl)-amino]-methyl}-benzyl)-O-methyl-hydroxylamine;
[2-Aminomethyl-4-(1H-imidazol-2-yl)-benzyl]-(1H-benzoimidazol-2-ylmethyl)-(5,6,7,8-tetrahydro-quinolin-8-yl)-amine;
[2-Aminomethyl-4-(1-methyl-1H-imidazol-2-yl)-benzyl]-(1H-benzoimidazol-2-ylmethyl)-(5,6,7,8-tetrahydro-quinolin-8-yl)-amine;
[2-Aminomethyl-4-(2H-pyrazol-3-yl)-benzyl]-(1H-benzoimidazol-2-ylmethyl)-(5,6,7,8-tetrahydro-quinolin-8-yl)-amine;
[2-Aminomethyl-4-(1-methyl-1H-pyrazol-3-yl)-benzyl]-(1H-benzoimidazol-2-ylmethyl)-(5,6,7,8-tetrahydro-quinolin-8-yl)-amine;
[2-Aminomethyl-4-(1H-[1,2,4]triazol-3-yl)-benzyl]-(1H-benzoimidazol-2-ylmethyl)-(5,6,7,8-tetrahydro-quinolin-8-yl)-amine;
[2-Aminomethyl-4-(1-methyl-1H-[1,2,4]triazol-3-yl)-benzyl]-(1H-benzoimidazol-2-ylmethyl)-(5,6,7,8-tetrahydro-quinolin-8-yl)-amine;
(2-Aminomethyl-4-oxazol-2-yl-benzyl)-(1H-benzoimidazol-2-ylmethyl)-(5,6,7,8-tetrahydro-quinolin-8-yl)-amine;
(2-Aminomethyl-4-furan-2-yl-benzyl)-(1H-benzoimidazol-2-ylmethyl)-(5,6,7,8-tetrahydro-quinolin-8-yl)-amine;
[2-Aminomethyl-4-(tetrahydro-furan-2-yl)-benzyl]-(1H-benzoimidazol-2-ylmethyl)-(5,6,7,8-tetrahydro-quinolin-8-yl)-amine;
(2-Aminomethyl-4-thiazol-2-yl-benzyl)-(1H-benzoimidazol-2-ylmethyl)-(5,6,7,8-tetrahydro-quinolin-8-yl)-amine;
[2-Aminomethyl-4-(1H-tetrazol-5-yl)-benzyl]-(1H-benzoimidazol-2-ylmethyl)-(5,6,7,8-tetrahydro-quinolin-8-yl)-amine;
[2-Aminomethyl-4-(2-methyl-2H-tetrazol-5-yl)-benzyl]-(1H-benzoimidazol-2-ylmethyl)-(5,6,7,8-tetrahydro-quinolin-8-yl)-amine;
(2-Aminomethyl-4-pyridin-2-yl-benzyl)-(1H-benzoimidazol-2-ylmethyl)-(5,6,7,8-tetrahydro-quinolin-8-yl)-amine;
(2-Aminomethyl-4-piperidin-2-yl-benzyl)-(1H-benzoimidazol-2-ylmethyl)-(5,6,7,8-tetrahydro-quinolin-8-yl)-amine;

(4-Aminomethyl-3-{[(1H-benzoimidazol-2-ylmethyl)-(5,6,7,8-tetrahydro-quinolin-8-yl)-amino]-methyl}-phenyl)-methanol;
(2-Aminomethyl-5-methoxymethyl-benzyl)-(1H-benzoimidazol-2-ylmethyl)-(5,6,7,8-tetrahydro-quinolin-8-yl)-amine;
(4-Aminomethyl-5-{[(1H-benzoimidazol-2-ylmethyl)-(5,6,7,8-tetrahydro-quinolin-8-yl)-amino]-methyl}-pyridin-2-yl)-methanol;
(4-Aminomethyl-6-methoxymethyl-pyridin-3-ylmethyl)-(1H-benzoimidazol-2-ylmethyl)-(5,6,7,8-tetrahydro-quinolin-8-yl)-amine;
(1H-Benzoimidazol-2-ylmethyl)-(4,6-bis-aminomethyl-pyridin-3-ylmethyl)-(5,6,7,8-tetrahydro-quinolin-8-yl)-amine;
(4-Allylaminomethyl-2-aminomethyl-benzyl)-(1H-benzoimidazol-2-ylmethyl)-(5,6,7,8-tetrahydro-quinolin-8-yl)-amine;
(2-Allylaminomethyl-4-aminomethyl-benzyl)-(1H-benzoimidazol-2-ylmethyl)-(5,6,7,8-tetrahydro-quinolin-8-yl)-amine;
(2-Aminomethyl-4-cyclopropylaminomethyl-benzyl)-(1H-benzoimidazol-2-ylmethyl)-(5,6,7,8-tetrahydro-quinolin-8-yl)-amine;
(4-Aminomethyl-2-cyclopropylaminomethyl-benzyl)-(1H-benzoimidazol-2-ylmethyl)-(5,6,7,8-tetrahydro-quinolin-8-yl)-amine;
(2-Aminomethyl-5-chloro-benzyl)-(1H-benzoimidazol-2-ylmethyl)-(5,6,7,8-tetrahydro-quinolin-8-yl)-amine;
(2-Aminomethyl-5-bromo-benzyl)-(1H-benzoimidazol-2-ylmethyl)-(5,6,7,8-tetrahydro-quinolin-8-yl)-amine;
(2-Aminomethyl-5-nitro-benzyl)-(1H-benzoimidazol-2-ylmethyl)-(5,6,7,8-tetrahydro-quinolin-8-yl)-amine;
4-Aminomethyl-3-{[(1H-benzoimidazol-2-ylmethyl)-(5,6,7,8-tetrahydro-quinolin-8-yl)-amino]-methyl}-benzonitrile;
(5-Amino-2-aminomethyl-benzyl)-(1H-benzoimidazol-2-ylmethyl)-(5,6,7,8-tetrahydro-quinolin-8-yl)-amine;
(2-Aminomethyl-5-trifluoromethyl-benzyl)-(1H-benzoimidazol-2-ylmethyl)-(5,6,7,8-tetrahydro-quinolin-8-yl)-amine;
(2-Aminomethyl-4-fluoro-benzyl)-(1H-benzoimidazol-2-ylmethyl)-(5,6,7,8-tetrahydro-quinolin-8-yl)-amine;
(2-Aminomethyl-4-chloro-benzyl)-(1H-benzoimidazol-2-ylmethyl)-(5,6,7,8-tetrahydro-quinolin-8-yl)-amine;
(2-Aminomethyl-4-bromo-benzyl)-(1H-benzoimidazol-2-ylmethyl)-(5,6,7,8-tetrahydro-quinolin-8-yl)-amine;
(2-Aminomethyl-4-nitro-benzyl)-(1H-benzoimidazol-2-ylmethyl)-(5,6,7,8-tetrahydro-quinolin-8-yl)-amine;
3-Aminomethyl-4-{[(1H-benzoimidazol-2-ylmethyl)-(5,6,7,8-tetrahydro-quinolin-8-yl)-amino]-methyl}-benzonitrile;
(4-Amino-2-aminomethyl-benzyl)-(1H-benzoimidazol-2-ylmethyl)-(5,6,7,8-tetrahydro-quinolin-8-yl)-amine;
(2-Aminomethyl-4-trifluoromethyl-benzyl)-(1H-benzoimidazol-2-ylmethyl)-(5,6,7,8-tetrahydro-quinolin-8-yl)-amine;
(4-Aminomethyl-2-fluoro-benzyl)-(1H-benzoimidazol-2-ylmethyl)-(5,6,7,8-tetrahydro-quinolin-8-yl)-amine;
(4-Aminomethyl-2-chloro-benzyl)-(1H-benzoimidazol-2-ylmethyl)-(5,6,7,8-tetrahydro-quinolin-8-yl)-amine;
(4-Aminomethyl-2-bromo-benzyl)-(1H-benzoimidazol-2-ylmethyl)-(5,6,7,8-tetrahydro-quinolin-8-yl)-amine;
(4-Aminomethyl-2-nitro-benzyl)-(1H-benzoimidazol-2-ylmethyl)-(5,6,7,8-tetrahydro-quinolin-8-yl)-amine;
5-Aminomethyl-2-{[(1H-benzoimidazol-2-ylmethyl)-(5,6,7,8-tetrahydro-quinolin-8-yl)-amino]-methyl}-benzonitrile;
(2-Amino-4-aminomethyl-benzyl)-(1H-benzoimidazol-2-ylmethyl)-(5,6,7,8-tetrahydro-quinolin-8-yl)-amine;
(4-Aminomethyl-2-trifluoromethyl-benzyl)-(1H-benzoimidazol-2-ylmethyl)-(5,6,7,8-tetrahydro-quinolin-8-yl)-amine;
(5-Aminomethyl-thiophen-2-ylmethyl)-(1H-benzoimidazol-2-ylmethyl)-(5,6,7,8-tetrahydro-quinolin-8-yl)-amine;
(4-Aminomethyl-thiophen-3-ylmethyl)-(1H-benzoimidazol-2-ylmethyl)-(5,6,7,8-tetrahydro-quinolin-8-yl)-amine;
(4-Aminomethyl-furan-3-ylmethyl)-(1H-benzoimidazol-2-ylmethyl)-(5,6,7,8-tetrahydro-quinolin-8-yl)-amine;
(4-Aminomethyl-1H-pyrrol-3-ylmethyl)-(1H-benzoimidazol-2-ylmethyl)-(5,6,7,8-tetrahydro-quinolin-8-yl)-amine;
(4-Aminomethyl-1-methyl-1H-pyrrol-3-ylmethyl)-(1H-benzoimidazol-2-ylmethyl)-(5,6,7,8-tetrahydro-quinolin-8-yl)-amine;
(4-Aminomethyl-1H-pyrazol-3-ylmethyl)-(1H-benzoimidazol-2-ylmethyl)-(5,6,7,8-tetrahydro-quinolin-8-yl)-amine;
(4-Aminomethyl-1-methyl-1H-pyrazol-3-ylmethyl)-(1H-benzoimidazol-2-ylmethyl)-(5,6,7,8-tetrahydro-quinolin-8-yl)-amine;
(3-Aminomethyl-1H-pyrazol-4-ylmethyl)-(1H-benzoimidazol-2-ylmethyl)-(5,6,7,8-tetrahydro-quinolin-8-yl)-amine;
(3-Aminomethyl-1-methyl-1H-pyrazol-4-ylmethyl)-(1H-benzoimidazol-2-ylmethyl)-(5,6,7,8-tetrahydro-quinolin-8-yl)-amine;
(5-Aminomethyl-3H-imidazol-4-ylmethyl)-(1H-benzoimidazol-2-ylmethyl)-(5,6,7,8-tetrahydro-quinolin-8-yl)-amine;
(5-Aminomethyl-1-methyl-1H-imidazol-4-ylmethyl)-(1H-benzoimidazol-2-ylmethyl)-(5,6,7,8-tetrahydro-quinolin-8-yl)-amine;
(5-Aminomethyl-thiazol-4-ylmethyl)-(1H-benzoimidazol-2-ylmethyl)-(5,6,7,8-tetrahydro-quinolin-8-yl)-amine;
(5-Aminomethyl-pyrimidin-4-ylmethyl)-(1H-benzoimidazol-2-ylmethyl)-(5,6,7,8-tetrahydro-quinolin-8-yl)-amine;
(5-Aminomethyl-pyridazin-4-ylmethyl)-(1H-benzoimidazol-2-ylmethyl)-(5,6,7,8-tetrahydro-quinolin-8-yl)-amine;
(5-Allylaminomethyl-2-{[(1H-benzoimidazol-2-ylmethyl)-(5,6,7,8-tetrahydro-quinolin-8-yl)-amino]-methyl}-phenyl)-methanol;
(3-Allylaminomethyl-4-{[(1H-benzoimidazol-2-ylmethyl)-(5,6,7,8-tetrahydro-quinolin-8-yl)-amino]-methyl}-phenyl)-methanol;
(4-Allylaminomethyl-2-methoxymethyl-benzyl)-(1H-benzoimidazol-2-ylmethyl)-(5,6,7,8-tetrahydro-quinolin-8-yl)-amine;
(3-Allylaminomethyl-4-methoxymethyl-benzyl)-(1H-benzoimidazol-2-ylmethyl)-(5,6,7,8-tetrahydro-quinolin-8-yl)-amine;
(2-{[((1H-Benzoimidazol-2-ylmethyl)-(5,6,7,8-tetrahydro-quinolin-8-yl)-amino]-methyl}-5-cyclopropylaminomethyl-phenyl)-methanol;
(4-{[((1H-Benzoimidazol-2-ylmethyl)-(5,6,7,8-tetrahydro-quinolin-8-yl)-amino]-methyl}-3-cyclopropylaminomethyl-phenyl)-methanol;
(1H-Benzoimidazol-2-ylmethyl)-(4-cyclopropylaminomethyl-2-methoxymethyl-benzyl)-(5,6,7,8-tetrahydro-quinolin-8-yl)-amine;
(1H-Benzoimidazol-2-ylmethyl)-(2-cyclopropylaminomethyl-4-methoxymethyl-benzyl)-(5,6,7,8-tetrahydro-quinolin-8-yl)-amine;
5-Aminomethyl-2-{[(1H-benzoimidazol-2-ylmethyl)-(5,6,7,8-tetrahydro-quinolin-8-yl)-amino]-methyl}-benzamide;
5-Aminomethyl-2- {[(1H-benzoimidazol-2-ylmethyl)-(5,6,7,8-tetrahydro-quinolin-8-yl)-amino]-methyl}-N-hydroxy-benzamide;
5-Aminomethyl-2-{[(1H-benzoimidazol-2-ylmethyl)-(5,6,7,8-tetrahydro-quinolin-8-yl)-amino]-methyl}-benzoic acid hydrazide;
5-Aminomethyl-2- {[(1H-benzoimidazol-2-ylmethyl)-(5,6,7,8-tetrahydro-quinolin-8-yl)-amino]-methyl}-benzoic acid;
(1H-Benzoimidazol-2-ylmethyl)-(2,4-bis-allylaminomethyl-benzyl)-(5,6,7,8-tetrahydro-quinolin-8-yl)-amine;
(4-Allylaminomethyl-2-cyclopropylaminomethyl-benzyl)-(1H-benzoimidazol-2-ylmethyl)-(5,6,7,8-tetrahydro-quinolin-8-yl)-amine;
(2-Allylaminomethyl-4-cyclopropylaminomethyl-benzyl)-(1H-benzoimidazol-2-ylmethyl)-(5,6,7,8-tetrahydro-quinolin-8-yl)-amine;
(1H-Benzoimidazol-2-ylmethyl)-(2,4-bis-cyclopropylaminomethyl-benzyl)-(5,6,7,8-tetrahydro-quinolin-8-yl)-amine;
(2-Aminomethyl-4-propyl-benzyl)-(1H-benzoimidazol-2-ylmethyl)-(5,6,7,8-tetrahydro-quinolin-8-yl)-amine;
(4-Allyl-2-aminomethyl-benzyl)-(1H-benzoimidazol-2-ylmethyl)-(5,6,7,8-tetrahydro-quinolin-8-yl)-amine;
Acetic acid 3-aminomethyl-4-{[(1H-benzoimidazol-2-ylmethyl)-(5,6,7,8-tetrahydro-quinolin-8-yl)-amino]-methyl}-benzyl ester;
Acetic acid 5-aminomethyl-2-{[(1H-benzoimidazol-2-ylmethyl)-(5,6,7,8-tetrahydro-quinolin-8-yl)-amino]-methyl}-benzyl ester;
Acetic acid 4- {[(1H-benzoimidazol-2-ylmethyl)-(5,6,7,8-tetrahydro-quinolin-8-yl)-amino]-methyl}-3-cyclopropylaminomethyl-benzyl ester;
Acetic acid 2-{[(1H-benzoimidazol-2-ylmethyl)-(5,6,7,8-tetrahydro-quinolin-8-yl)-amino]-methyl}-5-cyclopropylaminomethyl-benzyl ester;
Acetic acid 3-allylaminomethyl-4-{[(1H-benzoimidazol-2-ylmethyl)-(5,6,7,8-tetrahydro-quinolin-8-yl)-amino]-methyl}-benzyl ester;
Acetic acid 5-allylaminomethyl-2-{[(1H-benzoimidazol-2-ylmethyl)-(5,6,7,8-tetrahydro-quinolin-8-yl)-amino]-methyl}-benzyl ester;
5-Aminomethyl-2-{[(1H-benzoimidazol-2-ylmethyl)-(5,6,7,8-tetrahydro-quinolin-8-yl)-amino]-methyl}-benzaldehyde oxime;
3-Aminomethyl-4-{[(1H-benzoimidazol-2-ylmethyl)-(5,6,7,8-tetrahydro-quinolin-8-yl)-amino]-methyl}-benzaldehyde oxime;
N-(5-Aminomethyl-2-{[(1H-benzoimidazol-2-ylmethyl)-(5,6,7,8-tetrahydro-quinolin-8-yl)-amino]-methyl}-benzyl)-acetamide;
N-(3-Aminomethyl-4- {[(1H-benzoimidazol-2-ylmethyl)-(5,6,7,8-tetrahydro-quinolin-8-yl)-amino]-methyl}-benzyl)-acetamide;
N-(3-(Acetylamino-methyl)-4-{[(1H-benzoimidazol-2-ylmethyl)-(5,6,7,8-tetrahydro-quinolin-8-yl)-amino]-methyl}-benzyl)-acetamide;
N-(2- {[(1H-Benzoimidazol-2-ylmethyl)-(5,6,7,8-tetrahydro-quinolin-8-yl)-amino]-methyl}-benzyl)-acetamide;
(6-Aminomethyl-1,3-dihydro-isobenzofuran-5-ylmethyl)-(1H-benzoimidazol-2-ylmethyl)-(5,6,7,8-tetrahydro-quinolin-8-yl)-amine;
(4-Aminomethyl-1,3-dihydro-isobenzofuran-5-ylmethyl)-(1H-benzoimidazol-2-ylmethyl)-(5,6,7,8-tetrahydro-quinolin-8-yl)-amine;
(7-Aminomethyl-1,3-dihydro-isobenzofuran-4-ylmethyl)-(1H-benzoimidazol-2-ylmethyl)-(5,6,7,8-tetrahydro-quinolin-8-yl)-amine;
N'-(1*H*-benzimidazol-2-ylmethyl)-N'-(5,6,7,8-tetrahydro-8-quinolinyl)-1,3-benzenedimethanamine;
(1*H*-Benzimidazol-2-ylmethyl)-(2-Aminomethyl-benzyl)-(5,6,7,8-tetrahydro-quinolin-8-yl)-amine (hydrobromide salt);
(2-Aminomethyl-benzyl)-(1*H*-benzimidazol-2-ylmethyl)-(*S*)-5,6,7,8-tetrahydro-quinolin-8-yl-amine (hydrochloride salt);
(3-aminomethyl-4-{[(1*H*-benzoimidazol-2-ylmethyl)-(5,6,7,8-tetrahydro-quinolin-8-yl)-amino]-methyl}-phenyl)-methanol;
(2-Aminomethyl-3-methoxy-benzyl)-(1*H*-benzimidazol-2-ylmethyl)-(5,6,7,8-tetrahydro-quinolin-8-yl)-amine(hydrobromide salt);
(1*H*-Benzimidazol-2-ylmethyl)-(5,6,7,8-tetrahydroquinolin-8-yl)-[(1-aminomethyl)-benzoxazol-3-ylmethyl)]-amine (hydrobromide salt);
(1*H*-Benzimidazol-2-ylmethyl)-(5,6,7,8-tetrahydroquinolin-8-yl)-[(1-benzyl-2-aminomethyl)-imidazol-5-ylmethyl)]-amine;
6-aminomethylpyridin-3-ylmethyl-(1*H*-benzimidazol-2-ylmethyl)-(5,6,7,8-tetrahydroquinolin-8-yl)-amine;
[4-((2-amino-ethyl)-benzyl]-(1*H*-benzoimidazol-2-ylmethyl)-(5,6,7,8-tetrahydro-quinolin-8-yl)-amine (hydrobromide salt);
[4-((3-amino-propyl)-benzyl]-(1*H*-benzoimidazol-2-ylmethyl)-(5,6,7,8-tetrahydro-quinolin-8-yl)-amine (hydrobromide salt);
*N*-(4-{[(1*H*-benzimidazol-2-ylmethyl)-(5,6,7,8-tetrahydro-quinolin-8-yl)-amino]-methyl}-benzyl)-hydroxylamine;
(5-aminomethyl-2-{[(1*H*-benzoimidazol-2-ylmethyl)-(5,6,7,8-tetrahydro-quinolin-8-yl)-amino]-methyl}-phenyl)-methanol;
2-Aminomethyl-5-{[(1*H*-benzimidazol-2-ylmethyl)-(5,6,7,8-tetrahydro-quinolin-8-yl)-amino]-methyl}-phenol(hydrobromide salt);
(4-Aminomethyl-3-methoxy-benzyl)-(1*H*-benzimidazol-2-ylmethyl)-(5,6,7,8-tetrahydro-quinolin-8-yl)-amine (hydrobromide salt);
(1*H*-benzoimidazol-2-ylmethyl)-(2,4-bis-aminomethyl-benzyl)-(5,6,7,8-tetrahydro-quinolin-8-yl)-amine (hydrobromide salt);
5-Aminomethyl-2-{[(1*H*-benzoimidazol-2-ylmethyl)-(5,6,7,8-tetrahydro-quinolin-8-yl)-amino]-methyl}-benzoic acid methyl ester (hydrobromide salt);
3-aminomethyl-4-{[(1*H*-benzimidazole-2-ylmethyl)-(5,6,7,8-tetrahydro-quinolin-8-yl)-amino]-methyl}-benzoic acid hydrobromide salt;
3-aminomethyl-4-{[(1*H*-benzimidazole-2-ylmethyl)-(5,6,7,8-tetrahydro-quinolin-8-yl)-amino]-methyl}-*N*-hydroxy-benzamide hydrobromide salt;
3-aminomethyl-4-{[(1*H*-benzimidazole-2-ylmethyl)-(5,6,7,8-tetrahydro-quinolin-8-yl)-amino]-methyl}-benzamide hydrobromide salt;
3-Aminomethyl-4-{[(1*H*-benzimidazole-2-ylmethyl)-(5,6,7,8-tetrahydro-quinolin-8-yl)-amino]-methyl}-benzoic acid hydrazide (hydrobromide salt);
(2-aminomethyl-5-fluorobenzyl)-(1*H*-benzimidazol-2-ylmethyl)-(5,6,7,8-tetrahydro-quinolin-8-yl)-amine (hydrobromide salt);
3-aminomethyl-4-{[(1*H*-benzimidazol-2-ylmethyl)-(5,6,7,8-tetrahydro-quinolin-8-yl)-amino]-methyl}-benzoic acid methyl ester;
(2-aminomethyl-4-methoxymethyl-benzyl)-(1*H*-benzimidazol-2-ylmethyl)-(5,6,7,8-tetrahydro-quinolin-8-yl)-amine;
*N*-(2-{[(1*H*-benzimidazol-2-ylmethyl)-(5,6,7,8-tetrahydroquinolin-8-yl)-amino]-methyl}-benzyl)-guanidine;
*N*-(4-{[(1*H*-benzimidazol-2-ylmethyl)-(5,6,7,8-tetrahydroquinolin-8-yl)-amino]-methyl}-benzyl)-guanidine (hydrobromide salt);
*N'*-(4-{[(1*H*-Benzimidazol-2-ylmethyl)-(5,6,7,8-tetrahydro-quinolin-8-yl)-amino]-methyl}-benzyl)-*N*,*N*-dimethyl-guanidine (hydrobromide salt);
[4-((1*H*-benzimidazol-2-ylmethyl)-(5,6,7,8-tetrahydroquinolin-8-yl)-aminomethylbenzyl]-N,N-dimethylformamidine (hydrobromide salt);
*N*-(4-{[(1*H*-Benzimidazol-2-ylmethyl)-(5,6,7,8-tetrahydro-quinolin-8-yl)-amino]-methyl}-benzyl)-benzamidine (hydrobromide salt);
*N*-(4-{[(1*H*-Benzimidazol-2-ylmethyl)-(5,6,7,8-tetrahydro-quinolin-8-yl)-amino]-methyl}-benzyl)-acetamidine (hydrobromide salt);
N-isobutyl-N'-(1*H*-benzimidazol-2-ylmethyl)-N'-(5,6,7,8-tetrahydro-8-quinolinyl)-1,4-benzenedimethanamine (hydrobromide salt);
(1*H*-Benzimidazol-2-ylmethyl)-(4-piperidin-2-yl-benzyl)-(5,6,7,8-tetrahydro-quinolin-8-yl)-amine (hydrobromide salt);
(1*H*-Benzimidazol-2-ylmethyl)-(4-piperidin-1-ylmethyl-benzyl)-(5,6,7,8-tetrahydro-quinolin-8-yl)-amine (hydrobromide salt);
(1*H*-Benzimidazol-2-ylmethyl)-(4-methylaminomethyl-benzyl)-(5,6,7,8-tetrahydro-quinolin-8-yl)-amine (hydrobromide salt);
(1*H*-Benzimidazol-2-ylmethyl)-(4-piperazin-1-ylmethyl-benzyl)-(5,6,7,8-tetrahydro-quinolin-8-yl)-amine (hydrobromide salt);
[4-((4-Allyl-piperazin-1-ylmethyl)-benzyl]-(1*H*-benzimidazol-2-ylmethyl)-(5,6,7,8-tetrahydro-quinolin-8-yl)-amine (hydrobromide salt);
(1*H*-Benzimidazol-2-ylmethyl)-(4-dimethylaminomethyl-benzyl)-(5,6,7,8-tetrahydro-quinolin-8-yl)-amine (hydrobromide salt);
(1*H*-Benzimidazol-2-ylmethyl)-(5,6,7,8-tetrahydro-quinolin-8-yl)-[4-(1,2,4-triazol-4-yliminomethyl)-benzyl]-amine (hydrobromide salt);
*N'*-(4-{[(1*H*-Benzimidazol-2-ylmethyl)-(5,6,7,8-tetrahydro-quinolin-8-yl)-amino]-methyl}-benzyl)-ethane-1,2-diamine (hydrobromide salt);
(1*H*-benzimidazol-2-ylmethyl)-(4-butylaminomethyl-benzyl)-(5,6,7,8-tetrahydro-quinolin-8-yl)-amine;
(1*H-*benzimidazol-2-ylmethyl)-(4-diallylaminomethyl-benzyl)-(5,6,7,8-tetrahydro-quinolin-8-yl)-amine;
(4-allylaminomethyl-benzyl)-(1*H*-benzimidazol-2-ylmethyl)-(5,6,7,8-tetrahydro-quinolin-8-yl)-amine;
(1*H*-benzimidazol-2-ylmethyl)-(4-pyrrolidin-1-ylmethyl-benzyl)-(5,6,7,8-tetrahydro-quinolin-8-yl)-amine;
(1*H*-Benzimidazol-2-ylmethyl)-(4-morpholin-4-ylmethyl-benzyl)-(5,6,7,8-tetrahydro-quinolin-8-yl)-amine (hydrobromide salt);
(1*H*-benzoimidazol-2-ylmethyl)-(5,6,7,8-tetrahydro-quinolin-8-yl)-(4-thiomorpholin-4-ylmethyl-benzyl)-amine;
(1*H*-Benzimidazol-2-ylmethyl)-(5,6,7,8-tetrahydro-quinolin-8-yl)- (2-cyclopropylaminomethyl-benzyl)- amine (HBr salt);
(1*H*-Benzimidazol-2-ylmethyl)-(5,6,7,8-tetrahydro-quinolin-8-yl)- (2-allylaminomethyl-benzyl)- amine (HBr salt);
(1*H*-Benzimidazol-2-ylmethyl)-[2-(*R*)-(2-aminopropionamidylmethyl)-benzyl]-(5,6,7,8-tetrahydro-quinolin-8-yl)-amine (hydrobromide salt);
(1*H-*benzimidazol-2-ylmethyl)-[2-(1*H*-benzimidazol-2-ylmethyl)-aminobenzyl]-(5,6,7,8-tetrahydroquinolin-8-yl)-amine;
(2-aminobenzyl)-(1*H*-benzimidazol-2-ylmethyl)-(5,6,7,8-tetrahydroquinolin-8-yl)-amine;
(1*H*-Benzimidazol-2-ylmethyl)-(2-cyano-benzyl)-(5,6,7,8-tetrahydro-quinolin-8-yl)-amine (hydrobromide salt);
2-{[((1*H*-Benzimidazol-2-ylmethyl)-(5,6,7,8-tetrahydro-quinolin-8-yl)-amino]-methyl}-6-methoxy-benzoic acid ethyl ester (hydrobromide salt);
(6-aminopyridin-3-ylmethyl)-(benzimidazol-2-ylmethyl)-(5,6,7,8-tetrahydroquinolin-8-yl)-amine(hydrobromide salt);
(2-aminopyridin-3-ylmethyl)-(benzimidazol-2-ylmethyl)-(5,6,7,8-tetrahydro-8-quinolinyl)-amine (hydrobromide salt);
*N-*(4-{[(1*H*-Benzimidazol-2-ylmethyl)-(5,6,7,8-tetrahydro-quinolin-8-yl)-amino]-methyl}-phenyl)-guanidine (hydrobromide salt);
(4-Amino-benzyl)-(1*H*-benzoimidazol-2-ylmethyl)-(5,6,7,8-tetrahydro-quinolin-8-yl)-amine (hydrobromide salt);
N'-({[(1*H*-Benzimidazol-2-ylmethyl)-(5,6,7,8-tetrahydroquinolin-8-yl)-amino]-methyl}-phenyl)-N,N-dimethylformamidine;
4-{[((1*H*-Benzimidazol-2-ylmethyl)-(5,6,7,8-tetrahydro-quinolin-8-yl)-amino]-methyl}-benzaldehyde oxime;
[4-((1*H*-benzimidazol-2-ylmethyl)-(5,6,7,8-tetrahydroquinolin-8-yl)-aminomethyl]-benzamidine (hydrobromide salt);
4-{[((1*H*-Benzimidazol-2-ylmethyl)-(5,6,7,8-tetrahydro-quinolin-8-yl)-amino]-methyl}-benzyl alcohol;
4-{[((1*H*-Benzimidazol-2-ylmethyl)-(5,6,7,8-tetrahydro-quinolin-8-yl)-amino]-methyl}-benzaldehyde;
4-{[((1*H*-Benzimidazol-2-ylmethyl)-(5,6,7,8-tetrahydro-quinolin-8-yl)-amino]-methyl}-benzoic acid methyl ester;
(*R*,*S*)-4-{[(1*H*-Benzimidazol-2-ylmethyl)-(5,6,7,8-tetrahydro-quinolin-8-yl)-amino]-methyl}-*N*-hydroxy-benzamide;
4-{[((1*H*-Benzimidazol-2-ylmethyl)-(5,6,7,8-tetrahydro-quinolin-8-yl)-amino]-methyl}-benzoic acid hydrazide;
4-{[((1*H*-benzoimidazol-2-ylmethyl)-(5,6,7,8-tetrahydro-quinolin-8-yl)-amino]-methyl}-benzoic acid (hydrobromide salt);
4-{[((1*H*-Benzimidazol-2-ylmethyl)-(5,6,7,8-tetrahydro-quinolin-8-yl)-amino]-methyl}-benzamide;
(6-Amino-pyridin-2-ylmethyl)-(1*H*-benzimidazol-2-ylmethyl)-(5,6,7,8-tetrahydro-quinolin-8-yl)-amine (hydrobromide salt);
(2-{[((1*H*-Benzimidazol-2-ylmethyl)-(5,6,7,8-tetrahydro-quinolin-8-yl)-amino]-methyl}-phenyl)-methanol (free base);
*O*-(2-{[(1*H*-Benzimidazol-2-ylmethyl)-(5,6,7,8-tetrahydro-quinolin-8-yl)-amino]-methyl}-benzyl)-hydroxylamine (hydrobromide salt);
(4-Amino-pyridin-3-ylmethyl)-(1*H*-benzimidazol-2-ylmethyl)-(5,6,7,8-tetrahydro-quinolin-8-yl)-amine(hydrobromide salt);
2-{[((1*H*-Benzimidazol-2-ylmethyl)-(5,6,7,8-tetrahydro-quinolin-8-yl)-amino]-methyl}-5-cyano-benzoic acid methyl ester;
4-{[((1*H*-benzimidazol-2-ylmethyl)-(5,6,7,8-tetrahydroquinolin-8-yl)-amino]-methyl}-3-cyano-benzamide;
[3-((1*H*-benzimidazol-2-yl)-benzyl]-(1*H*-benzimidazol-2-ylmethyl)-(5,6,7,8-tetrahydroquinolin-8-yl)-amine (hydrobromide salt);
(1*H*-Benzimidazol-2-ylmethyl)-(5,6,7,8-tetrahydroquinolin-8-yl)-(imidazol-2-yl)-methylamine (hydrobromide salt);
4-{[((1*H*-Benzimidazol-2-ylmethyl)-(5,6,7,8-tetrahydro-quinolin-8-yl)-amino]methyl}-2,6-dichloropyridine (hydrobromide salt);
(1*H*-benzoimidazol-2-ylmethyl)-benzooxazol-5-ylmethyl-(5,6,7,8-tetrahydro-quinolin-8-yl)-amine;
pyridin-2-ylmethyl-(1*H*-benzimidazol-2-ylmethyl)-(5,6,7,8-tetrahydroquinolin-8-yl)-amine;
(1*H*-benzimidazol-2-ylmethyl)-benzoxazol-6-ylmethyl-(5,6,7,8-tetrahydro-quinolin-8-yl)-amine;
(1*H*-benzimidazol-4-ylmethyl)-(1*H*-benzimidazol-2-ylmethyl)-(5,6,7,8-tetrahydro-quinolin-8-yl)-amine (hydrobromide salt);
(1*H*-Benzimidazol-2-ylmethyl)-pyridin-4-ylmethyl-(5,6,7,8-tetrahydro-quinolin-8-yl)-amine;
(1*H*-Benzimidazol-2-ylmethyl)-(benzo[1,3]dioxol-4-ylmethyl)-(5,6,7,8-tetrahydro-quinolin-8-yl)-amine;
benzo[1,3]dioxol-5-ylmethyl-(1*H*-benzoimidazol-2-ylmethyl)-(5,6,7,8-tetrahydro-quinolin-8-yl)-amine;
(1*H*-Benzimidazol-2-ylmethyl)-(2,3-dihydro-benzofuran-7-ylmethyl)-(5,6,7,8-tetrahydro-quinolin-8-yl)-amine (hydrobromide salt);
(1*H*-Benzimidazol-2-ylmethyl)-pyridin-3-ylmethyl-(5,6,7,8-tetrahydro-quinolin-8-yl)-amine (hydrobromide salt);
(1*H*-benzoimidazol-5-ylmethyl)-(1*H*-benzoimidazol-2-ylmethyl)-(5,6,7,8-tetrahydro-quinolin-8-yl)-amine (hydrobromide salt);
Bis-(1*H*-benzimidazol-2-ylmethyl)-(5,6,7,8-tetrahydro-quinolin-8-yl)-amine (hydrobromide salt);
(1*H*-Benzimidazol-2-ylmethyl)-(3*H*-imidazol-4-ylmethyl)-(5,6,7,8-tetrahydro-quinolin-8-yl)-amine (hydrobromide salt);
[4-((1*H*-benzimidazol-2-yl)-benzyl]-(1*H*-benzimidazol-2-ylmethyl)-(5,6,7,8-tetrahydroquinolin-8-yl)-amine (hydrobromide salt);
(1*H*-Benzimidazol-2-ylmethyl)-(4-pyrid-2-yl-benzyl)-(5,6,7,8-tetrahydro-quinolin-8-yl)-amine (hydrobromide salt);
(1*H*-Benzimidazol-2-ylmethyl)-[4-(oxazol-2-yl)-benzyl]-(5,6,7,8-tetrahydroquinolin-8-yl)-amine (hydrobromide salt);
(1*H*-Benzimidazol-2-ylmethyl)-(4-imidazol-1-yl-benzyl)-(5,6,7,8-tetrahydro-quinolin-8-yl)-amine (hydrobromide salt);
[4-((thiazol-2-yl)-benzyl]-(1*H*-benzimidazol-2-ylmethyl)-(5,6,7,8-tetrahydroquinolin-8-yl)-amine (hydrobromide salt);
(1*H*-Benzimidazol-2-ylmethyl)-[4-(benzothiazol-2-yl)-benzyl]-(5,6,7,8-tetrahydroquinolin-8-yl)-amine (hydrobromide salt);
[4-((benzoxazol-2-yl)-benzyl]-(1*H*-benzimidazol-2-ylmethyl)-(5,6,7,8-tetrahydroquinolin-8-yl)-amine (hydrobromide salt);
[4-((1*H*-imidazol-2-yl)-benzyl]-(1*H*-benzimidazol-2-ylmethyl)-(5,6,7,8-tetrahydroquinolin-8-yl)-amine (hydrobromide salt);
(2'-Aminomethyl-biphenyl-4-ylmethyl)-(1*H*-benzimidazol-2-ylmethyl)-(5,6,7,8-tetrahydro-quinolin-8-yl)-amine (hydrobromide salt);
(1*H*-Benzimidazol-2-ylmethyl)-(2'-methoxy-biphenyl-4-ylmethyl)-(5,6,7,8-tetrahydro-quinolin-8-yl)-amine (hydrobromide salt);
(1*H*-Benzimidazol-2-ylmethyl)-(4-oxazol-5-yl-benzyl)-(5,6,7,8-tetrahydro-quinolin-8-yl)-amine (hydrobromide salt);
(1*H*-Benzimidazol-2-ylmethyl)-(5,6,7,8-tetrahydro-quinolin-8-yl)-(4-thiophen-2-yl-benzyl)-amine (hydrobromide salt);
(1*H*-Benzimidazol-2-ylmethyl)-[4-(2-methyl-2*H*-tetrazol-5-yl)-benzyl]-(5,6,7,8-tetrahydro-quinolin-8-yl)-amine (hydrobromide salt); and
(1*H*-Benzimidazol-2-ylmethyl)-[4-(5-phenyloxazol-2-yl)-benzyl]-(5,6,7,8-tetrahydroquinolin-8-yl)-amine.

Methods to synthesize the compounds useful in the method of the invention are set forth in the U.S. patents and applications incorporated hereinabove by reference.

As provided above, AMD3100 is an antagonist with the CXCR4 chemokine receptor (Gerlach, et al., J.Biol. Chem. (2001) 276:14153-14160). This compound interferes with the binding of bone marrow stromal cell derived SDF-1 with CXCR4 on stem cells which leads to the release of hematopoietic stem cells from bone marrow into the circulation (Broxmeyer, et al., Blood (2001) 98:811a (Abstract)). In a Phase 1 study at the University of Washington, Seattle, a single dose of 80 µg/kg of AMD-3100 resulted in a WBC count of 17,000/µl and a peak 6-fold increase in circulating CD34+ progenitor/stem cells at the 6 hour time point (Liles, et al., Blood 2001 98:737a (Abstract)).

In another recent study, mice were injected with rhG-CSF and recombinant rat Stem Cell Factor (rrSCF) in order to mobilize large numbers of bone marrow stem cells into the circulation and then we induced a heart attack. The combination of rrSCF and rhG-CSF provides a peak number of circulating stem cells after 5 daily injections. At 27 days post surgery there was a 68% improvement in survival in the treated group versus the controls. At this time the dead tissue was replaced with regenerating myocardium and all functional parameters tested were improved compared with controls (Orlic, et al., PNAS (2001) 98:10344-10349).

The compounds useful for the methods of the invention may be prepared in the form of prodrugs, *i.e.,* protected forms which release the compounds of the invention after administration to the subject. Typically, the protecting groups are hydrolyzed in body fluids such as in the bloodstream thus releasing the active compound or are oxidized or reduced in *vivo* to release the active compound. A discussion of prodrugs is found in Smith and Williams Introduction to the Principles of Drug Design, Smith, H.J.; Wright, 2nd ed., London (1988).

The polyamine compounds useful for the methods of the invention may be administered prepared in the forms of their acid addition salts or metal complexes thereof. Suitable acid addition salts include salts of inorganic acids that are biocompatible, including HCl, HBr, sulfuric, phosphoric and the like, as well as organic acids such as acetic, propionic, butyric and the like, as well as acids containing more than one carboxyl group, such as oxalic, glutaric, adipic and the like. Typically, at physiological pH, the compounds of the invention will be in the forms of the acid addition salts. Particularly preferred are the hydrochlorides. In addition; when prepared as purified forms, the compounds may also be crystallized as the hydrates.

The compounds useful for the methods of the invention may be administered as sole active ingredients, as mixtures of various compounds of formula (1), and/or in admixture with additional active ingredients that are therapeutically or nutritionally useful, such as antibiotics, vitamins, herbal extracts, anti-inflammatories, glucose, antipyretics, analgesics, granulocyte-macrophage colony stimulating factor (GM-CSF), Interleukin-1 (IL-1), Interleukin-3 (IL-3), Interleukin-8 (IL-8), PIXY-321 (GM-CSF/IL-3 fusion protein), macrophage inflammatory protein, stem cell factor, thrombopoietin, growth related oncogene or chemotherapy and the like.

The compounds useful for the methods of the invention may be formulated for administration to an animal subject using commonly understood formulation techniques well known in the art. Formulations which are suitable for particular modes of administration and for compounds of the type represented by those of formula (1) may be found in Remington's Pharmaceutical Sciences, latest edition, Mack Publishing Company, Easton, PA.

In one embodiment, the compounds are administered by injection. For example, the compounds may be administered by intravenous, subcutaneous or intraperitoneal injection, and the like. Additional parenteral routes of administration include intramuscular and intraarticular injection. For intravenous or parenteral administration, the compounds are formulated in suitable liquid form with excipients as required. The compositions may contain liposomes or other suitable carriers. For injection intravenously, the solution is made isotonic using standard preparations such as Hank's solution.

Besides injection, other routes of administration may be used. The compounds may be formulated into tablets, capsules, syrups, powders, or other suitable forms for administration orally. By using suitable excipients, these compounds may also be administered through the mucosa using suppositories or intranasal sprays. Transdermal administration can also be effected by using suitable penetrants and controlling the rate of release.

The formulation and route of administration chosen will be tailored to the individual subject, the nature of the condition to be treated in the subject, and generally, the judgment of the attending practitioner.

Suitable dosage ranges for the compounds of formula (1) vary according to these considerations, but in general, the compounds are administered in the range of about 0.1 µg/kg-5 mg/kg of body weight; preferably the range is about 1 µg/kg-300 µg/kg of body weight; more preferably about 10 µg/kg-100 µg/kg of body weight. For a typical 70-kg human subject, thus, the dosage range is from about 0.7 µg-350 mg; preferably about 700 µg-21 mg; most preferably about 700 µg-7 mg. Dosages may be higher when the compounds are administered orally or transdermally as compared to, for example, i.v. administration.

The compounds may be administered as a single bolus dose, a dose over time, as in i.v. or transdermal administration, or in multiple dosages.

The compounds for use in the methods of the present invention may also be used in *ex vivo* treatment protocols to prepare cell cultures which are then directly administered to damaged tissues of the subjects. *Ex vivo* treatment can be conducted on autologous cells harvested from the peripheral blood or bone marrow or from allografts from matched donors. The concentration of the compound or compounds of formula (1)-(3), alone or in combination with other agents, such as macrophage inflammatory protein is a matter of routine optimization.

Various methods for direct administration to tissues are known in the art. For example, direct administration to tissues can be accomplished using cathether-based methods (*e.g*., infusion catheter, stiletto catheter, or balloon catheters), stents, needles, needle-free injectors, channeling devices, or other appropriate medical device for direct administration to a tissue. Alternatively, surgical approaches such as via open chest or thorascoscopy and surgical patches may be used for direct administration to a tissue, such as the myocardium.

Subjects that will respond favorably to the method of the invention include medical and veterinary subjects generally, including human patients. Among other subjects for whom the methods of the invention is useful are rats, mouse, cats, dogs, large animals, avians such as chickens, and the like. In general, any subject who would benefit from an elevation of progenitor cells and/or stem cells, or whose progenitor cells and/or stem cells are desirable for stem cell transplantation are appropriate for administration of the invention method.

In one embodiment, mobilized progenitor and/or stem cells are directly administered to damaged tissues in a subject who has suffered or is suffering from cardiovascular disease, arteriosclerosis, stroke, congestive heart failure, myocardial infarct, myocardial ischemia, or angina. The subject may also be a diabetic subject, who commonly suffer cardiovascular complications, including peripheral vascular diseases (Schatteman, et al., Journal of Clinical Investigation, 2000, vol., 106 no. 4, p.571).

The damaged tissue may be a damaged organ tissue in the myocardium, limbs, brain, and liver. Other subjects that may benefit from the methods of the invention include subjects who have a hematopoietic disorder, such as aplastic anemia, leukemias, drug-induced anemias, and hematopoietic deficits from chemotherapy or radiation therapy.

### Refence Example 1

### Elevation of Mouse Progenitor Cell Levels

The effects of subcutaneous (s.c.) administration of 1,1'-[1,4-phenylene-bis(methylene)]-bis-1,4,8,11-tetraazacyclotetradecane (AMD3100) to C3H/H3 J mice on numbers of granulocyte macrophage (CFU-GM), erythroid (BFU-E), and multipotential (CFU-GEMM) progenitor cells per mL of blood were measured. Progenitors were stimulated to form colonies *in vitro* with the combination of 1U/m rhu Epo, 50 ng/ml rhu SLF, 5% ^{Vol}/_{Vol} pokeweed mitogen mouse spleen cell conditioned medium (PWMSCM), and 0.1 mM hemin. Plates were scored 7 days after incubation.

The time dependent effects on the number of progenitors mobilized with AMD3100 are for a single s.c. injection of 5 mg/Kg and are shown in Table 1.

**Table 1**

| | **Absolute Progenitors Per ML Blood Methylcellulose Culture** | | |
|---|---|---|---|
| | **CFU-GM** | **BFU-E** | **CFU-GEMM** |
| **Control** | 289.8 | 49.4 | 25.8 |
| **AMD3100: 15"** | 791.6 | 134.5 | 90.4 |
| **AMD3100: 30"** | 1805.5 | 209.3 | 113.5 |
| **AMD3100: 120"** | 828.7 | 102.3 | 47.6 |

To measure the dose-dependent effects, AMD3100 was administered at 1, 2.5, 5 and 10 mg/Kg via a single s.c. injection and the number of progenitors per mL of blood was measured at 1 hour post administration, and the results are shown in Table 2.

**Table 2**

| | **Absolute Number Progenitors Per ML Blood Methylcellulose Culture** | | |
|---|---|---|---|
| | **CFU-GM** | **BFU-E** | **CFU-GEMM** |
| **Saline** | 188.1 | 16 | 19 |
| **AMD3100**: **10mg/kg** | 825.6 | 120.5 | 79.8 |
| **AMD3100: 5mg/kg** | 608.4 | 92.8 | 69.5 |
| **AMD3100: 2.5mg/kg** | 687.6 | 98.9 | 70.6 |
| **AMD3100: 1mg/kg** | 424 | 62 | 27.1 |

| | **Fold Change Compared to Time 0** | | |
|---|---|---|---|
| | **Progenitors Methylcellulose Culture** | | |
| **Time** | **GM** | **BFU-E** | **CFU-GEMM** |
| 15" | 2.73 | 2.72 | 3.51 |
| 30" | 6.23 | 4.24 | 4.41 |
| 2' | 2.86 | 2.07 | 1.85 |

Maximum mobilization of mouse progenitors is achieved at a dose of 2.5 to 10 mg/kg AMD3100, approximately 0.5 to 1 hour after injection, as shown in Table 3.

### Refence Example 2

### Mobilization of Mouse Progenitor Cells in Combination with MIP-1α and G-CSF

The progenitor cell mobilization capacity of AMD3100 in combination with mouse (mu) macrophage inflammatory protein (MIP-1α) was tested with or without prior administration of rhu G-CSF. MIP-1α has been previously shown to mobilize progenitor cells in mice and humans (Broxmeyer, H. E., et al., Blood Cells, Molecules, and Diseases (1998) 24(2):14-30).

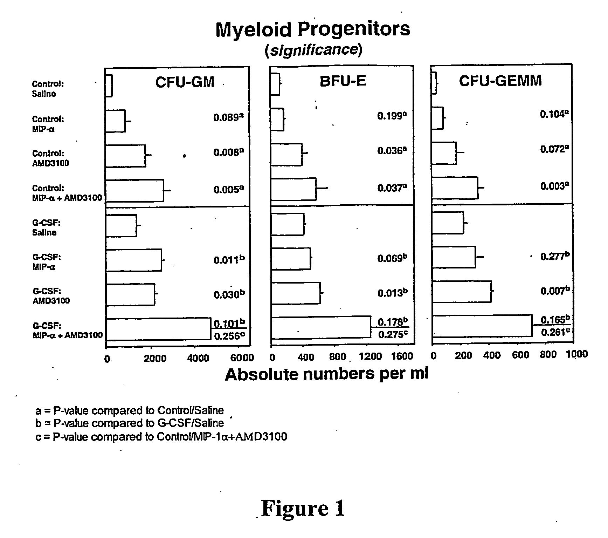

Groups of mice were randomized to receive control diluent (saline) or G-CSF at a dose of 2.5 µg per mouse, twice a day, for two days via s.c. injection. Eleven hours after the final injection of saline or G-CSF, the mice were divided into groups to receive MIP-1α administered I.V. at a total dose of 5 µg, AMD3100 administered s.c. at a dose of 5 mg/Kg, or a combination of both MIP-1α and AMD3100 at the same doses. One hour later, the mice were sacrificed and the number of progenitor cells per mL of blood were measured. These data are summarized in Figure 1.

AMD3100 acts in an additive to greater than additive manner for mobilization of progenitor cells when used in combination with mouse (mu) macrophage inflammatory protein (MIP)-1α, each given 11 hours after the addition of rhu G-CSF or control diluent (saline) and 1 hour prior to assessing the blood.

### Refence Example 3

### Clinical Elevation of Progenitor Cell Levels

Five healthy human volunteers having initial white blood cell counts of 4,500-7,500 cells/mm³ were used in the study. Each patient was given a single subcutaneous (s.c.) injection of 80 µg/kg AMD3100 (i.e., 1,1'-[1,4-phenylene-bis(methylene)]-bis-1,4,8,11-tetraazacyclotetradecane) in 0.9% saline, from a stock solution of 10 mg/mL AMD3100 in saline, under sterile conditions. Blood samples were obtained via catheter prior to the dose, and at various times up to 24 hours after dosing.

The blood samples were evaluated for total white blood cells, CD34 positive progenitor cells (via FACS analysis) as a percentage of total white blood cells, as well as the absolute numbers per mL and cycling status of granulocyte macrophage (CFU-GM), erythroid (BFU-E), and multipotential (CFU-GEMM) progenitor cells.

As shown in Tables 3 and 4, administration of AMD3100 caused an elevation of the white blood cell count and of CD34 positive progenitor cells in human volunteers which maximized at 6 hours post-administration.

**Table 3**

| AMD3100 induced mobilization of white blood cells in individual volunteers (x 10³ WBC's). | | | | | | | | | |
|---|---|---|---|---|---|---|---|---|---|
| ID | Screen | Baseline | TREATMENT | | | | | | |
| | | | 30 Min | 1 Hr | 2 Hr | 4 Hr | 6 Hr | 9 Hr | Day 2 |
| P1 | 7.4 | 6.41 | 8.02 | 14.8 | 21.4 | 23.2 | 26.2 | 22.3 | 7.07 |
| P2 | 6.04 | 5.45 | 6.53 | 8.93 | 13.5 | 18.00 | 19.2 | 19.6 | 8.03 |
| P3 | 4.38 | 5.8 | 7.14 | 9.28 | ND | 18.10 | 17.9 | 18.4 | 4.98 |
| P4 | 5.08 | 5.31 | 4.37 | 7.38 | 12.4 | 14.6 | 15.8 | 13.9 | 4.98 |
| P5 | 4.53 | 5.02 | 6.08 | 8.43 | ND | 16.90 | 19.3 | 19.00 | 4.57 |

**Table 4**

| AMD3100 induced mobilization of CD34 positive cells, expressed as the percentage of the total WBC's in individual volunteers. | | | | | | |
|---|---|---|---|---|---|---|
| ID | Baseline | TREATMENT | | | | |
| | | 1 Hr | 3 Hr | 6 Hr | 9 Hr | Day 2 |
| P1 | .07 | .04 | .07 | .11 | .11 | .08 |
| P2 | .08 | .06 | .08 | .13 | .11 | .12 |
| P3 | .07 | .16 | .06 | ND | .11 | .07 |
| P4 | .05 | .07 | .09 | .09 | .1 | .1 |
| P5 | .12 | .12 | .13 | .2 | .2 | .16 |

The blood was also analyzed for AMD3100 mobilized these progenitors.

Absolute numbers of unseparated and low density (Fico-hypaque separated) nucleated cells per ml of blood, as well as the absolute numbers per ml and cycling status of granulocyte macrophage (CFU-GM), erythroid (BFU-E), and multipotential (CFU-GEMM) progenitor cells were measured in normal donors injected s.c. with AMD3100. The above parameters were assessed prior to injection and at 1, 3, 6, 9 and 24 hours after injection of AMD3100. All progenitor cell results are based on the scoring of 3 culture plates per assay per point.

For the progenitor cell numbers and cycling status, the numbers of CFU-GM, BFU-E and CFU-GEMM in methylcellulose cultures by stimulation of the cells with 1 Unit (U)/ml recombinant human (rhu) erythropoietin, 100 U/ml rhu granulocytemacrophage colony stimulating factor (GM-CSF), 100 U/ml rhu interleukin-3 (IL-3) and 50 ng/ml rhu steel factor (SLF = stem cell factor (SCF)). The CFU-GM were also evaluated in agar cultures stimulated with 100 U/ml rhu GM-CSF and 50 ng/ml rhu SLF. For both types of assays, colonies were scored after 14 day incubation in a humidified atmosphere with 5% CO₂ and lowered (5%) O₂ tension. Cell cycling status of progenitors was measured using a high specific activity tritiated thymidine kill technique as previously described (Broxmeyer, H. E., et al., Exp. Hematol. (1989) 17:455-459).

The results are given first, as the mean fold change in absolute numbers of nucleated cells and progenitors at 1, 3, 6, 9 and 24 hours compared to the preinjection (=Time (T) 0) counts for all five donors, as seen in Tables 5-7.

In the tables below,
STD - Standard deviation
STE - Standard error
PBL-US - peripheral blood-unseparated
PBL-LD - peripheral blood-low density (Ficoll Separated)
P - Significance using a 2 tailed t test

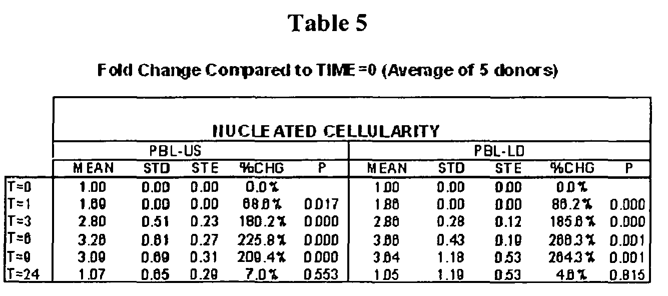

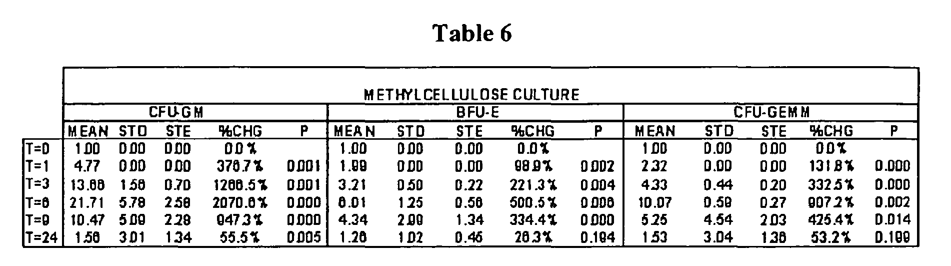

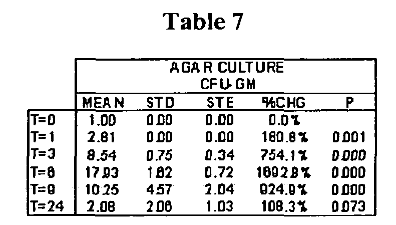

The results are then shown as a fold change from T=0 levels for each individual donor, as shown in Tables 8-10.

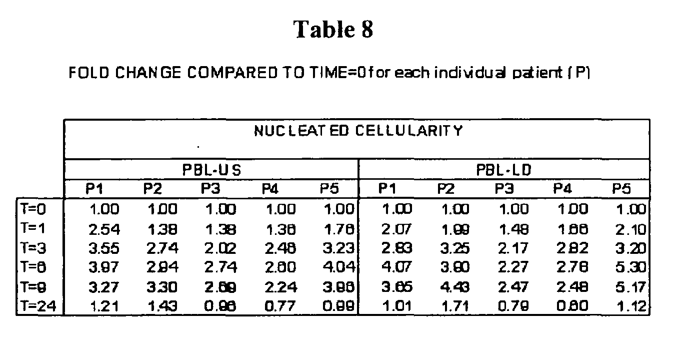

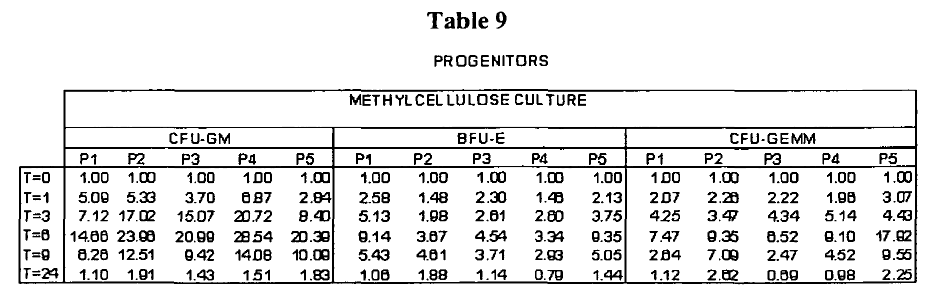

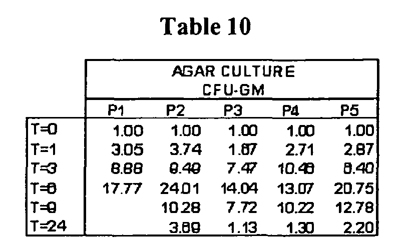

The actual nucleated cell and progenitor cell numbers per ml of blood and the cycling status (= % progenitors in DNA synthesis (S) phase of the cell cycle) of progenitors for each of the five donors (#'s P1, P2, P3, P4, and P5) is shown in Tables 11 and 12.

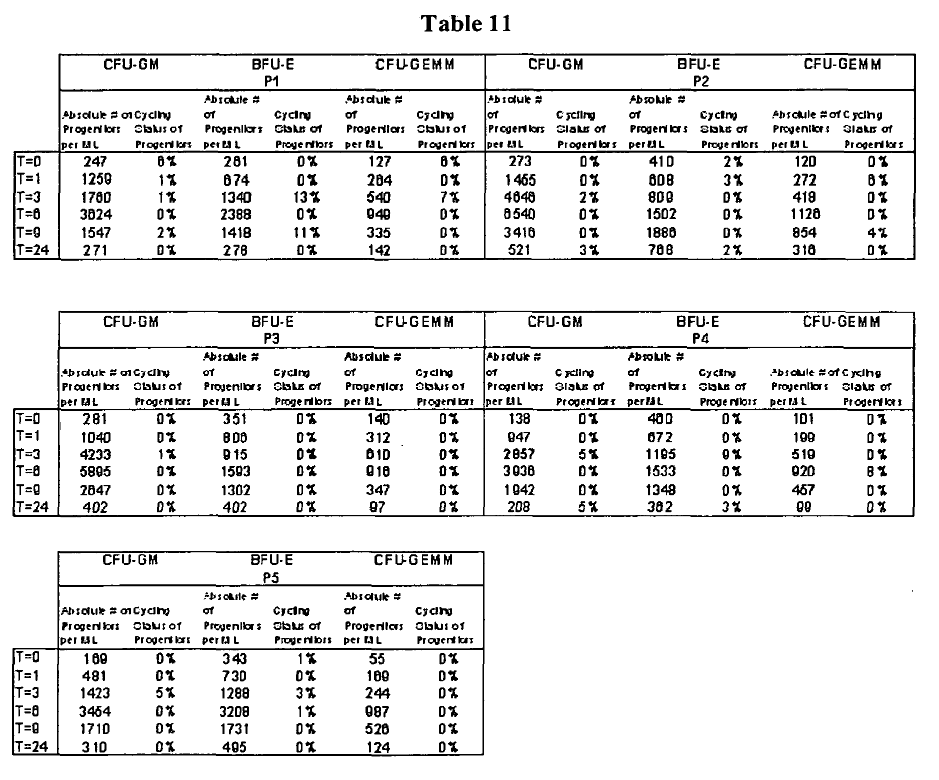

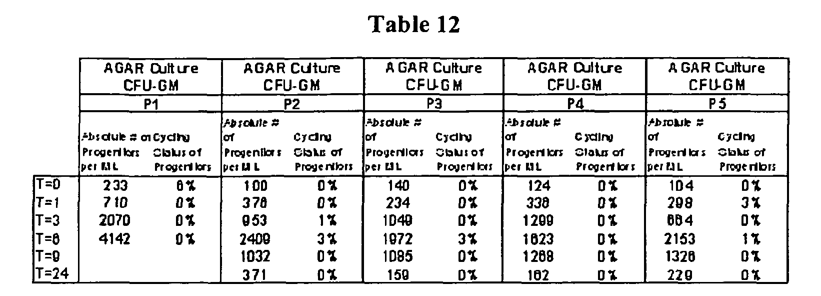

The results for all five donors were very consistent with maximal fold increases in circulating levels of progenitor cells seen 6 hours after injection of AMD3100 into the human donor subjects. Progenitors were in a slow or non-cycling state prior to and 1, 3, 6, 9 and 24 hours after injection of AMD3100.

### Refence Example 4

### Mobilized Bone Marrow Stem Cells for Myocardial Repair

Adult rats are anesthetized and a thoracotomy is performed. The descending branch of the left coronary artery is ligated and not reperfused. Within 4 to 6 hours after ligation the animals are injected with limit dilution AMD-3100 or AMD-3100 plus rhG-CSF. Control rats are not treated with the reagents. The animals are monitored at one-week intervals by echocardiography and MRI. The experiment is terminated at 2, 6 to 12 weeks post-surgery. On the day of sacrifice, the hemodynamic functions are analyzed for left ventricle-end diastolic pressure, left ventricle-developed pressure and the rate of rise and fall of left ventricle pressure. The heart is then arrested in diastole and perfused via the abdominal aorta to flush residual blood from the vascular network of the myocardium. This is followed by perfusion of the heart with 10% formalin. Several slices are made through the fixed heart and these are embedded in paraffin and sections. The sections are stained and analyzed by light microscopy to determine the size of the infarct in the treated and control animals. Tissue sections from hearts taken at 2 weeks after surgery are stained with antibodies specific for immature, developing myocyte and blood vessel proteins and analyzed by confocal microscopy. The immunohistochemical analysis involves the identification of transcription factors and surface markers expressed in early stages of myocyte development. The results of this experiment will show that when the reagent AMD-3100 is administered within hours after induction of cardiac ischemia, together with or without rhG-CSF, this reagent mobilizes bone marrow stem cells rapidly, and will result in a block to cardiac remodeling and scar formation and will lead to regeneration of the dead myocardium.

### Example 5

### Tissue Regeneration in Diabetic Animal Ischemic Model

Diabetes was chemically induced in male nude Hfh11 mice using streptozotocin (STZ). Three to four weeks after injection of STZ the left hind limb was made ischemic by ligation of the iliac artery and removal of a 0.5-1 cm segment distal to the ligation.

AMD3100 was administered intraperitoneally with 5 mg/kg of a 1.25mg/ml solution of AMD3100 (n=8) or an equal volume of normal saline (n=12) on the day of and two days after surgery. One group of additional mice (n=11) was injected intramuscularly into the ischemic hindlimb with human non-diabetic-derived unmobilized CD34+ (unCD34+) peripheral blood mononuclear cells (PBMCs), two to four hours after surgery. Human non-diabetic-derived unmobilized CD34+ are potent stimulators of vessel growth in diabetic mice, and may have similar properties in diabetic patients (Schatteman et al., J. Clin. Invest. (2000) 106:571-578). Another group of additional mice (n=7) was injected intramuscularly with AMD3100 mobilized CD34+ (mCD34+) human PBMCs (1x 10⁶), which were harvested by apheresis from human volunteers four hours after subcutaneous injection with 240 ug/kg of AMD3100. Untreated mice served as controls (n=10).

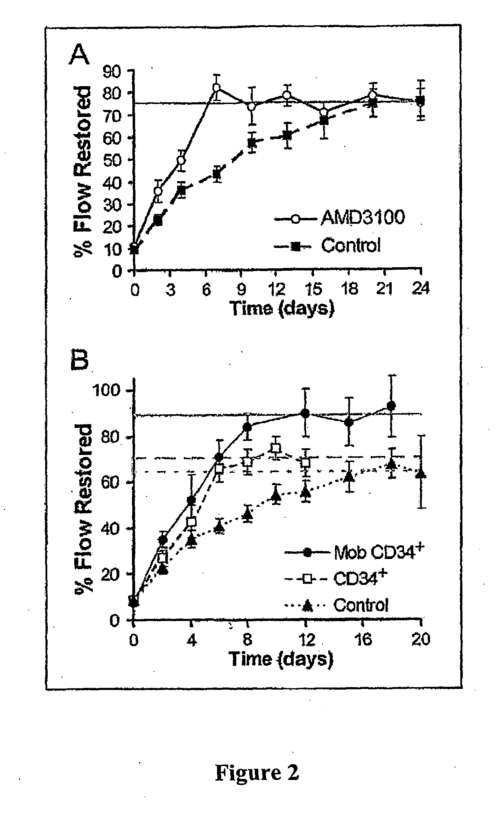

Limb blood flow restoration was analyzed immediately before and after surgery and at various times thereafter in the entire limb distal to the ligation using LASER Doppler analysis. Figure 2A shows the percent of blood flow restored over time in mice injected intraperitoneally with AMD 3100 or vehicle (control) on days 0 and 2. Gray line indicates plateau of flow restoration (mean flow d8-d24) in AMD3100 treated mice. As shown in Figure 2A, drug induced improvement was observed two days after surgery, and by seven days blood flow had reached its maximum in AMD3100 treated mice (Figure 2A, P<0.05). Thereafter, blood flow was maintained at a mean of 74.6 ± 2.8% that of the initial flow. Not until 16 days after surgery did flow in the control mice reach that of AMD3100 treated mice.

Furthermore, mCD34+ or unCD34+ PBMCs were injected directly into ischemic limb muscle to assess if AMD3100 mCD34+ PBMC therapy improves limb revascularization and if the cells are as potent as their unmobilized counterparts (*i.e*., unCD34+). Figure 2B shows the percent of blood flow restored over time in mice injected with AMD3100 mobilized CD34+ or unmobilized CD34+ PBMCs into the ischemic muscle on the day of ligation. Gray lines indicate plateau of flow restoration using mean flow for d15-20 in untreated (lower, dotted), d8-d12 in unmobilized CD34+ PBMC treated (middle, dashed), and d12-d18 in mobilized CD34+ cell treated (solid,upper) mice.

As shown in Figure 2B, both mCD34+ and unCD34+ cell types significantly accelerated blood flow restoration relative to untreated controls by 6 days after injection (Figure 2B, P<0.05). By 8 days after surgery blood flow values plateaued in unCD34+ PBMC injected limbs, maintaining a mean of 70.7 ± 3.9% of initial flow thereafter. Flow in limbs of control mice plateaued at a similar level (64.7 ± 4.6%) but did not reach this level until 15 days. In contrast, flow in mCD34+ PBMC treated limbs continued to improve until day 12, after which blood flow plateaued at 89.5 ± 6.6% of initial flow, a flow that was significantly greater than that of either control or unCD34+ PBMC treated limbs.

The results demonstrate that systemic treatment with AMD3100 (Figure 2A) and local injection of AMD3100 mCD34+ PBMCs (Figure 2B) accelerate blood flow restoration to ischemic tissue in a diabetic environment with similar kinetics. AMD3100 systemic therapy accelerated blood flow restoration, but did not increase it. In contrast, mCD34+ PBMC therapy both accelerated and increased blood flow restoration, and only mCD34+ PBMCs increased the amount of flow covered in the ischemic limbs relative to untreated controls.

It is understood that the foregoing detailed description and accompanying examples are merely illustrative, and are not to be taken as limitations upon the scope of the invention.

## Claims

1. The use of a compound having formula (1) for the manufacture of a medicament for increasing blood flow in an ischemic tissue, wherein said compound having formula (1) mobilizes progenitor and/or stem cells, which are harvested and directly administered to an ischemic tissue in a subject, wherein said compound is of the formula:
Z-linker-Z' (1)
or a pharmaceutically acceptable salt thereof,
wherein Z is a cyclic polyamine containing 9-32 ring members of which 2-8 are nitrogen atoms, said nitrogen atoms separated from each other by at least 2 carbon atoms, and wherein said heterocycle may optionally contain additional heteroatoms besides nitrogen and/or may be fused to an additional ring system;
Z' may be embodied in a form as defined by Z above, or alternatively may be of the formula:
-N(R)-(CR₂)ₙ-X
wherein each R is independently H or straight, branched or cyclic alkyl (1-6C),
n is 1 or 2, and
X is an aromatic ring, including heteroaromatic rings, or is a mercaptan;
"linker" represents a bond, alkylene (1-6C) or may comprise aryl, fused aryl, oxygen atoms contained in an alkylene chain or may contain keto groups or nitrogen or sulfur atoms.

2. Use of claim 1 wherein said compound of formula (1) is 1,1'-1,4-phenylene-bis-(methylene)-bis-1,4,8,11-tetraazacyclotetradecane, or a pharmaceutically acceptable salt thereof.

3. Use of claim 2, wherein said salt is a hydrochloride salt.

4. Use of any preceding claim, comprising treatment of cell populations ex vivo with a compound of formula (1) and directly administering the treated populations into damaged tissues in a compatible subject.

5. A compound having formula (1):
Z-linker-Z' (1)
or a pharmaceutically acceptable salt thereof,
wherein Z is a cyclic polyamine containing 9-32 ring members of which 2-8 are nitrogen atoms, said nitrogen atoms separated from each other by at least 2 carbon atoms, and wherein said heterocycle may optionally contain additional heteroatoms besides nitrogen and/or may be fused to an additional ring system;
Z' may be embodied in a form as defined by Z above, or alternatively may be of the formula
-N(R)-(CR₂)ₙ-X
wherein each R is independently H or straight, branched or cyclic alkyl (1-6C),
n is 1 or 2, and
X is an aromatic ring, including heteroaromatic rings, or is a mercaptan; "linker" represents a bond, alkylene (1-6C) or may comprise aryl, fused aryl, oxygen atoms contained in an alkylene claim, or may contain keto groups or nitrogen or sulfur atoms;
wherein said compound having formula (1) mobilizes progenitor and/or stem cells,
for use in a method, wherein the mobilized progenitor and/or stem cells are harvested and directly administered to an ischemic tissue in a subject to increase blood flow in an ischemic tissue.

6. A compound for use according to claim 5, further comprising the feature of any of claims 2 to 4.

## Patentansprüche

1. Verwendung einer Verbindung der Formel (1) für die Herstellung eines Medikamentes zur Erhöhung des Blutflusses in einem ischämischen Gewebe, wobei die Verbindung der Formel (1) Progenitor- und/oder Stammzellen mobilisiert, die geerntet werden und direkt an ein ischämisches Gewebe in einem Subjekt verabreicht werden, wobei die Verbindung die Formel:
Z-Linker-Z' (1)
hat
oder ein pharmazeutisch annehmbares Salz davon ist, wobei Z ein cyclisches Polyamin ist, das 9-32 Ringglieder enthält, von denen 2-8 Stickstoffatome sind, wobei die Stickstoffatome durch wenigstens 2 Kohlenstoffatome voneinander getrennt sind und wobei der Heterocyclus gegebenenfalls außer Stickstoff zusätzliche Heteroatome enthalten kann und/oder an ein weiteres Ringsystem kondensiert sein kann;
Z' in einer Form, wie sie durch Z oben definiert ist, verkörpert werden kann, oder alternativ die Formel:
-N(R)-(CR₂)ₙ-X
haben kann,
worin jedes R unabhängig H oder geradkettiges, verzweigtes oder cyclisches Alkyl (1-6C) ist,
n 1 oder 2 ist und
X ein aromatischer Ring, einschließlich heteroaromatischer Ringe, ist oder ein Mercaptan ist;
"Linker" eine Bindung, Alkylen (1-6C) darstellt oder Aryl, kondensiertes Aryl, Sauerstoffatome, die in einer Alkylenkette enthalten sind, umfassen kann oder Ketogruppen oder Stickstoff- oder Schwefelatome enthalten kann.

2. Verwendung gemäß Anspruch 1, wobei die Verbindung der Formel (1) 1,1'-1,4-Phenylen-bis(methylen)-bis-1,4,8,11-tetraazacyclotetradecan oder ein pharmazeutisch annehmbares Salz davon ist.

3. Verwendung gemäß Anspruch 2, wobei das Salz ein Hydrochloridsalz ist.

4. Verwendung gemäß einem vorangehenden Anspruch, umfassend Behandlung von Zellpopulationen ex vivo mit einer Verbindung der Formel (1) und direktes Verabreichen der behandelten Populationen in geschädigte Gewebe in einem kompatiblen Subjekt.

5. Verbindung der Formel (1):
Z-Linker-Z' (1)
oder ein pharmazeutisch annehmbares Salz davon,
wobei Z ein cyclisches Polyamin ist, das 9-32 Ringglieder enthält, von denen 2-8 Stickstoffatome sind, wobei die Stickstoffatome durch wenigstens 2 Kohlenstoffatome voneinander getrennt sind und wobei der Heterocyclus gegebenenfalls außer Stickstoff zusätzliche Heteroatome enthalten kann und/oder an ein weiteres Ringsystem kondensiert sein kann;
Z' in einer Form, wie sie durch Z oben definiert ist, verkörpert werden kann, oder alternativ die Formel:
-N(R)-(CR₂)ₙ-X
haben kann,
worin jedes R unabhängig H oder geradkettiges, verzweigtes oder cyclisches Alkyl (1-6C) ist,
n 1 oder 2 ist und
X ein aromatischer Ring, einschließlich heteroaromatischer Ringe, ist oder ein Mercaptan ist;
"Linker" eine Bindung, Alkylen (1-6C) darstellt oder Aryl, kondensiertes Aryl, Sauerstoffatome, die in einer Alkylenkette enthalten sind, umfassen kann oder Ketogruppen oder Stickstoff- oder Schwefelatome enthalten kann;
wobei die Verbindung der Formel (1) Progenitor- und/oder Stammzellen mobilisiert,
zur Verwendung in einem Verfahren, in dem die mobilisierten Progenitor- und/oder Stammzellen geerntet werden und direkt an ein ischämisches Gewebe in einem Subjekt verabreicht werden, um den Blutfluss in einem ischämischen Gewebe zu erhöhen.

6. Verbindung zur Verwendung gemäß Anspruch 5, die außerdem das Merkmal gemäß einem der Ansprüche 2 bis 4 umfasst.

## Revendications

1. Utilisation d'un composé de formule (1) pour la fabrication d'un médicament conçu pour augmenter le débit sanguin dans un tissu ischémique, ledit composé de formule (1) mobilisant des cellules progénitrices et/ou des cellules souches, qui sont prélevées et directement administrées au niveau d'un tissu ischémique chez un sujet, lequel composé a pour formule
Z-linker-Z' (1)
ou d'un sel pharmacologiquement admissible d'un tel composé,
dans laquelle formule
- Z représente une polyamine cyclique comportant de 9 à 32 chaînons de cycle dont 2 à 8 atomes d'azote, lesquels atomes d'azote sont séparés les uns des autres par au moins 2 atomes de carbone, et lequel hétérocycle peut, en option, comporter des hétéroatomes supplémentaires, en plus des atomes d'azote, et/ou être condensé avec un système cyclique supplémentaire ;
- Z' peut soit représenter une entité telle que définie ci-dessus pour Z, soit représenter une entité de formule
-N(R)-(CR₂)ₙ-X
dans laquelle
- chaque symbole R représente indépendamment un atome d'hydrogène ou un groupe alkyle en C₁-C₆, à chaîne linéaire, ramifiée ou cyclique,
- l'indice n vaut 1 ou 2,
- et X représente un cycle aromatique, y compris les cycles hétéro-aromatiques, ou représente une entité de type mercaptan ;
- et "linker" représente une liaison, un groupe alcanediyle en C₁-C₆, ou une entité comportant un groupe aryle, un groupe aryle à cycles condensés ou des atomes d'oxygène au sein d'une chaîne de type alcanediyle, ou une entité comportant des groupes carbonyle ou des atomes d'azote ou de soufre.

2. Utilisation conforme à la revendication 1, dans laquelle ledit composé de formule (1) est le 1,1'-[1,4-phénylène-bis(méthylène)-bis-(1,4,8,11-tétraaza-cyclotétradécane)] ou un sel pharmacologiquement admissible de ce composé.

3. Utilisation conforme à la revendication 2, dans laquelle ledit sel est un chlorhydrate.

4. Utilisation conforme à l'une des revendications précédentes, comprenant le fait de traiter *ex vivo* des populations de cellules avec un composé de formule (1), et le fait d'administrer directement ces populations traitées dans des tissus endommagés, chez un sujet compatible.

5. Composé de formule (1) :
Z-linker-Z' (1)
ou sel pharmacologiquement admissible d'un tel composé,
dans laquelle formule
- Z représente une polyamine cyclique comportant de 9 à 32 chaînons de cycle dont 2 à 8 atomes d'azote, lesquels atomes d'azote sont séparés les uns des autres par au moins 2 atomes de carbone, et lequel hétérocycle peut, en option, comporter des hétéroatomes supplémentaires, en plus des atomes d'azote, et/ou être condensé avec un système cyclique supplémentaire ;
- Z' peut soit représenter une entité telle que définie ci-dessus pour Z, soit représenter une entité de formule
-N(R)-(CR₂)ₙ-X
dans laquelle
- chaque symbole R représente indépendamment un atome d'hydrogène ou un groupe alkyle en C₁-C₆, à chaîne linéaire, ramifiée ou cyclique,
- l'indice n vaut 1 ou 2,
- et X représente un cycle aromatique, y compris les cycles hétéro-aromatiques, ou représente une entité de type mercaptan ;
- et "linker" représente une liaison, un groupe alcanediyle en C₁-C₆, ou une entité comportant un groupe aryle, un groupe aryle à cycles condensés ou des atomes d'oxygène au sein d'une chaîne de type alcanediyle, ou une entité comportant des groupes carbonyle ou des atomes d'azote ou de soufre ;
lequel composé de formule (1) mobilise des cellules progénitrices et/ou des cellules souches,
pour utilisation dans un procédé dans lequel les cellules progénitrices et/ou les cellules souches mobilisées sont prélevées et directement administrées au niveau d'un tissu ischémique chez un sujet, pour augmenter le débit sanguin dans un tissu ischémique.

6. Composé pour utilisation conforme à la revendication 5, auquel s'applique en outre la caractéristique de l'une des revendications 2 à 4.
